(19) Europäisches Patentamt
European Patent Office
Office européen des brevets

(11) Veröffentlichungsnummer: **0 459 285 B1**

(12) # EUROPÄISCHE PATENTSCHRIFT

(45) Veröffentlichungstag der Patentschrift: **14.12.94**

(51) Int. Cl.$^{5}$: **C07C 69/74**, C07C 69/743, C07C 251/48, A01N 53/00, A01N 37/50, A01N 37/36

(21) Anmeldenummer: **91108247.7**

(22) Anmeldetag: **22.05.91**

Die Akte enthält technische Angaben, die nach dem Eingang der Anmeldung eingereicht wurden und die nicht in dieser Patentschrift enthalten sind.

(54) **Ortho-substituierte Benzylester von Cyclopropancarbonsäuren.**

(30) Priorität: **31.05.90 DE 4017488**
**30.07.90 DE 4024094**

(43) Veröffentlichungstag der Anmeldung:
**04.12.91 Patentblatt 91/49**

(45) Bekanntmachung des Hinweises auf die Patenterteilung:
**14.12.94 Patentblatt 94/50**

(84) Benannte Vertragsstaaten:
**AT BE CH DE DK ES FR GB GR IT LI NL SE**

(56) Entgegenhaltungen:
**EP-A- 0 103 957**
**EP-A- 0 310 954**
**EP-A- 0 354 571**

(73) Patentinhaber: **BASF Aktiengesellschaft**
**Carl-Bosch-Strasse 38**
**D-67063 Ludwigshafen (DE)**

(72) Erfinder: **Schuetz, Franz, Dr.**
**Budapester Strasse 45**
**D-6700 Ludwigshafen (DE)**
Erfinder: **Sauter, Hubert, Dr.**
**Neckarpromenade 20**
**D-6800 Mannheim 1 (DE)**
Erfinder: **Goetz, Norbert, Dr.**
**Schoefferstrasse 25**
**D-6520 Worms 1 (DE)**
Erfinder: **Wild, Jochen, Dr.**
**St.-Martin-Strasse 22**
**D-6701 Ruppertsberg (DE)**
Erfinder: **Wolf, Hans-Josef, Dr.**
**Fichtestrasse 7**
**D-6701 Maxdorf (DE)**
Erfinder: **Doetzer, Reinhard, Dr.**
**Buchenweg 3**
**D-6940 Weinheim (DE)**
Erfinder: **Lorenz, Gisela, Dr.**
**Erlenweg 13**
**D-6730 Neustadt (DE)**
Erfinder: **Ammermann, Eberhard, Dr.**
**Sachsenstrasse 3**
**D-6700 Ludwigshafen (DE)**
Erfinder: **Kuenast, Christoph, Dr.**
**Salierstrasse 2**
**D-6701 Otterstadt (DE)**
Erfinder: **Kardorff, Uwe, Dr.**
**D 3,4**
**D-6800 Mannheim 1 (DE)**

EP 0 459 285 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue ortho-substituierte Benzylester von Cyclopropancarbonsäuren der allgemeinen Formel I

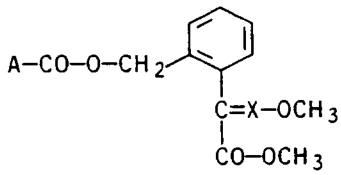

I

in der X für Stickstoff oder =CH- und A für den folgenden Cyclopropanreste steht:

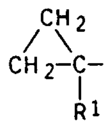

,

wobei der Substituent $R^1$ die folgende Bedeutung hat:

Cyano, $C_2$-$C_8$-Alkyl, Trifluormethyl, $C_3$-$C_8$-Alkenyl, Trimethylsilyl, Phenyl-$C_1$-$C_6$-alkyl oder Phenyl-$C_3$-$C_6$-alkenyl, wobei der Aromat jeweils noch 1-5 Halogenatome oder bis zu 3 der folgenden Substituenten tragen kann:

$C_1$-$C_6$-Alkyl, partiell oder vollständig halogeniertes $C_1$-$C_6$-Alkyl oder $C_1$-$C_6$-Alkoxy, $R^1$ bedeutet außerdem noch

2-Bromphenyl, 4-Trifluormethylphenyl, 2,4-Difluorphenyl, 2,6-Difluorphenyl oder 2-Fluor-6-chlorphenyl, mit der Maßgabe, daß X für =CH- steht, wenn $R^1$ Trifluormethyl oder Trimethylsilyl bedeutet.

Außerdem betrifft die Erfindung ein Verfahren zur Herstellung dieser Verbindungen, ihre Verwendung als Fungizide und ihre Verwendung zur Bekämpfung von Schädlingen sowie fungizide Mittel und Schädlingsbekämpfungsmittel, welche diese Verbindungen als wirksame Substanzen enthalten.

Aus der EP-A 310 954 sind unter anderem fungizid wirksame ortho-substituierte Benzylester von Cyclopropancarbonsäuren vom Typ der Verbindungen I bekannt, deren Cyclopropanring unsubstituiert ist oder einen Methyl-, Dichlor-Phenylrest oder einen verschieden substituierten Phenylrest trägt. Ferner sind aus der EP-A 354 571 u.a. 2-[1-(Trifluormethyl)-cyclopropylcarbonyloxymethyl]-phenyl- und 2-[1-(Trimethylsilyl)-cyclopropylcarbonyloxymethyl]-phenyl-glyoxylsäuremethylester-O-methyloxim bekannt. Eine insektizide Wirkung ist aus den genannten Druckschriften nicht bekannt.

Außerdem sind in EP-A 103 957 substituierte Benzylester mit pestiziden Eigenschaften beschrieben, welche in 3-Position eine Alkoxyiminogruppe tragen können.

Der Erfindung lagen neue ortho-substituierte Benzylester von Cyclopropancarbonsäuren als Aufgabe zugrunde.

Demgemäß wurden die eingangs definierten ortho-substituierten Benzylester von Cyclopropancarbonsäuren der Formel I gefunden.

Im einzelnen können die Substituenten in den erfindungsgemäßen Verbindungen I z.B. die folgenden Bedeutungen haben:

$R^1$

- Cyano;
- verzweigtes oder unverzweigtes $C_2$-$C_8$-Alkyl, insbesondere $C_2$-$C_6$-Alkyl wie Methyl, Ethyl, Isopropyl, n-Butyl, tert.-Butyl, n-Pentyl, Isopentyl und Neopentyl;
- verzweigtes oder unverzweigtes $C_3$-$C_8$-Alkenyl, insbesondere $C_3$-$6_6$-Alkenyl wie Prop-2-enyl, 1-Methyl-2-propenyl, But-2-enyl, 3-Methyl-but-2-enyl und 2-Methyl-prop-2-enyl;
- Phenyl-$C_1$-$C_6$-alkyl oder Phenyl-$C_3$-$C_6$-alkenyl, insbesondere Phenyl-$C_1$-$C_4$-alkyl oder Phenyl-$C_3$-$C_4$-alkenyl wie Benzyl, 2-Phenylethyl, 3-Phenyl-n-propyl, 4-Phenyl-n-butyl, 3-Phenylallyl und 4-Phenyl-2-butenyl,

  wobei der Aromat jeweils noch 1-5 Halogenatome wie Fluor, Chlor und Brom oder bis zu 3 der folgenden Substituenten tragen kann:

- verzweigtes oder unverzweigtes $C_1$-$C_6$-Alkyl, insbesondere $C_1$-$C_4$-Alkyl wie Methyl, Ethyl, n-Propyl, Isopropyl, n-Butyl, sec.-Butyl und tert.-Butyl,
- partiell oder vollständig halogeniertes, verzweigtes oder unverzweigtes $C_1$-$C_6$-Alkyl, insbesondere $C_1$-$C_4$-Alkyl wie Difluormethyl, Trifluormethyl, Trichlormethyl, Pentafluorethyl und 2-Chlor-1,1,2-trifluorethyl,
- verzweigtes oder unverzweigtes $C_1$-$C_6$-Alkoxy, insbesondere $C_1$-$C_4$-Alkoxy wie Methoxy, Ethoxy, n-Propoxy, Isopropoxy, n-Butoxy und tert.-Butoxy;
- 2-Bromophenyl;
- 4-Trifluormethylphenyl;
- 2,4- oder 2,6-Difluorphenyl;
- 2-Fluor-6-chlorphenyl;
- Trimethylsilyl oder Trifluormethyl.

Besonders geeignete Verbindungen I sind Tabelle 1 zu entnehmen. Bevorzugt werden Verbindungen I, deren Cyclopropanring durch folgende Reste $R^{1'}$ monosubsituiert ist:

- Cyano;
- $C_2$-$C_4$-Alkyl wie Ethyl, n-Propyl, Isopropyl und tert.-Butyl;
- Trifluormethyl;
- $C_3$-$C_4$-Alkenyl wie Prop-2-enyl und But-2-enyl;
- Phenyl-$C_1$-$C_4$-alkyl und Phenyl-$C_3$-$C_4$-alkenyl, wobei der Aromat jeweils noch einen der folgenden Substituenten tragen kann: Halogen wie Fluor, Chlor und Brom, oder Methyl, insbesondere Benzyl, 3-, 4-Fluorbenzyl, 3-, 4-Chlorbenzyl, 4-Brombenzyl, 3-Methylbenzyl, 3-(4-Fluorphenyl)-propyl, 3-(4-Chlorphenyl)-propyl, 3-(4-Fluorphenyl)-prop-2-enyl und 3-(4-Chlorphenyl)-prop-2-enyl;
- 2-Bromphenyl;
- 2,4- und 2,6-Difluorphenyl;
- 2-Fluor-6-chlorphenyl und
- Trimethylsilyl.

Bedeutet $R^{1'}$ eine Phenylalkenylgruppe, so ist die E-Konfiguration an der Doppelbindung besonders bevorzugt.

Die erfindungsgemäßen Verbindungen I können bei der Herstellung aufgrund des zum Benzylteil ortho-ständigen Substituenten $-C(COOCH_3)=X-OCH_3$ als E/Z-Isomerengemische anfallen. Die Isomeren können gewünschtenfalls nach den hierfür üblichen Methoden, z.B. durch Kristallisation oder Chromatographie getrennt werden. Verbindungen mit E-Konfiguration sind besonders bevorzugt. Die Gemische und die einzelnen Isomeren sind gut wirksam und werden von der Erfindung umfaßt.

Die ortho-substituierten Benzylester von Cyclopropancarbonsäuren I sind auf verschiedene Weise erhältlich, und zwar vorzugsweise dadurch, daß man eine Cyclopropancarbonsäure II in an sich bekannter Weise, in einem inerten Lösungsmittel wie Ethanol, mit einer Base in das Carboxylat-Anion überführt und dieses Anion mit einer ortho-substituierten Benzylverbindung III umsetzt (vgl. Synthesis 1975, 805):

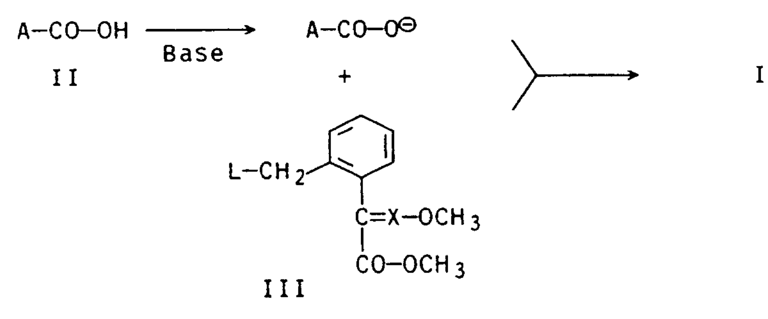


L bedeutet eine nucleophile Abgangsgruppe, insbesondere einen Sulfonyl-Rest wie Methansulfonyl, Trifluormethylsulfonyl, p-Toluolsulfonyl, p-Bromphenylsulfonyl oder einen Methylsulfat-Rest, bes. bevorzugt ein Halogenatom wie Chlor, Brom und Jod.

Als Basen sind besonders Alkalimetallhydroxide wie Natrium- und Kaliumhydroxid und Triethylamin geeignet.

Zweckmäßigerweise nimmt man die Umsetzung des Carboxylat-Anions mit der Benzylverbindung III in einem Lösungs- bzw. Verdünnungsmittel wie Aceton, Acetonitril, Dimethylsulfoxid, Dioxan, Dimethylformamid, N-Methylpyrrolidon, N,N'-Dimethylpropylenharnstoff und Pyridin oder, unter Verwendung eines Phasentransferkatalysators, in einem 2-Phasensystem aus Wasser und einem Kohlenwasserstoff wie Tetrachlorkohlenstoff vor.

Als Phasentransferkatalysatoren eignen sich z.B. Trioctylpropylammoniumchlorid und Cetyltrimethylammoniumchlorid (vgl. Synthesis 1974, 867).

Vorteilhaft setzt man alle Ausgangsverbindungen in etwa stöchiometrischem Verhältnis ein, jedoch kann in manchen Fällen auch ein Überschuß der einen oder anderen Komponente, etwa bis zu 10 %, empfehlenswert sein.

Im allgemeinen liegt die Reaktionstemperatur zwischen 0 ° C und der Siedetemperatur des Lösungsmittels, bevorzugt zwischen 20 und 130 ° C.

Da die Reaktion nicht druckabhängig ist, arbeitet man vorteilhaft bei Normaldruck.

Die Cyclopropancarbonsäuren II sind bekannt oder können nach an sich bekannten Verfahren hergestellt werden [siehe z.B. Synthesis, 738 (1987); Zh. Org. Khim 16, 2086 (1980); J. Am. Chem. Soc. 106, 6642 (1984); Chem. Ber. 116, 3895 (1983); Helv. Chim. Acta 69, 1655 (1986); Chem. Ber. 119, 3694 (1986); Chem. Letters, 475 (1989); J. Organometal. Chem. 46, 73 (1972); Gazz. Chim. Ital. 100, 566 (1970); J. Org. Chem. 48, 2472 (1983) und J. Org. Chem. 47, 893 (1982)].

Die ortho-substituierten Benzylverbindungen III (X = CH, L = Chlor oder Brom) sind ebenfalls bekannt oder können nach bekannten Verfahren hergestellt werden (vgl. z.B. DE-A 35 19 280, DE-A 35 45 318 und DE-A 35 45 319).

Beispielsweise kann das ortho-substituierte Benzylbromid III (X = CH, L = Brom) durch Bromierung des entsprechenden ortho-substituierten Toluols IV mit N-Bromsuccinimid erhalten werden (vgl. Angew. Chem. 71, 349 (1959):

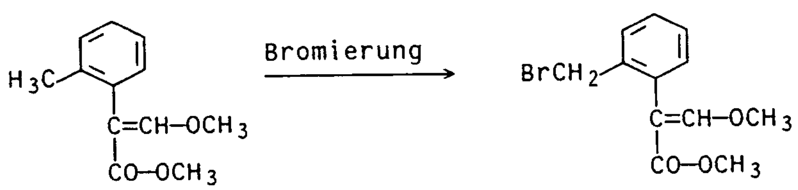


IV III

(X = CH, L = Br) Das ortho-substituierte Toluol IV ist herstellbar, indem man z.B. ein Hydroxymethylenderivat Va, das im Gleichgewicht mit dem entsprechenden Formylderivat Vb vorliegt, in Gegenwart einer Base wie Kaliumcarbonbat alkyliert:

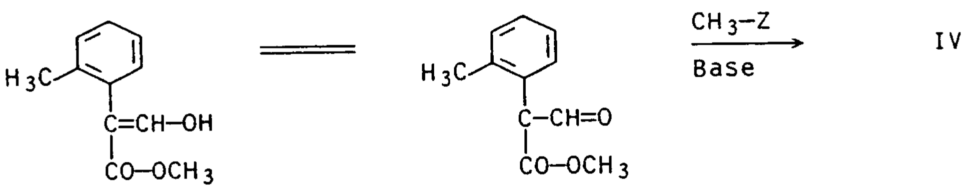


Va Vb

Z = Methylsulfat, Chlorid, Bromid, Jodid

Als Alkylierungsmittel ist beispielsweise Dimethylsulfat, Methyljodid, -chlorid und -bromid geeignet.

Normalerweise arbeitet man in einem inerten Lösungsmittel, beispielsweise in Aceton.

Die Reaktionstemperatur liegt im allgemeinen zwischen 20 und 60 ° C.

Das Hydroxymethylenderivat Va ist beispielsweise durch basenkatalysierte Umsetzung von 2-Methylphenylessigsäuremethylester mit Ameisensäuremethylester in einem inerten Lösungsmittel wie Diethylether und Tetrahydrofuran erhältlich, wobei als Base z.B. Natriumhydrid verwendet werden kann (vgl. Ann. Chem. 424, 214 (1921)).

Ortho-substituierte Benzylverbindungen III, wobei X Stickstoff und L Chlor oder Brom bedeutet, sind auf verschiedene Weise erhältlich, und zwar vorteilhaft durch Halogenierung des 2-Methylphenylglyoxylsäuremethylester-O-methyloxims VI.

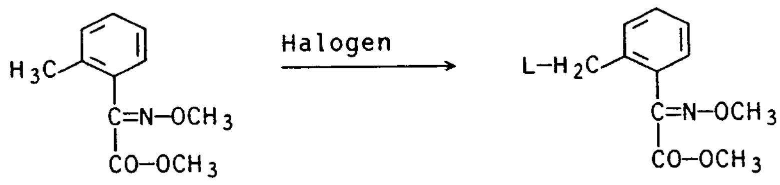


VI III

(X = N, L = Cl, Br) Zur Halogenierung können Chlor oder Brom in einem inerten Lösungsmittel, z.B. Tetrachlormethan, oder Halogenierungsmittel wie N-Chlor- und N-Bromsuccinimid [vgl. Angew. Chem. 71, 349 (1959)] in $CCl_4$ verwendet werden. Bei Verwendung von elementarem Chlor oder Brom empfiehlt es sich, die Reaktion photochemisch durch Bestrahlung mit Sonnenlicht durchzuführen.

Das aus der DE-A 36 23 921 bekannte 2-Methylphenylglyoxylsäuremethylester-O-methyloxim VI ist vorteilhaft aus dem 2-Methyl-phenylglyoxylsäuremethylester erhältlich, durch Umsetzung mit O-Methylhydroxylamin-hydrochlorid oder mit Hydroxylaminhydrochlorid, wobei in diesem Fall primär ein Oxim entsteht, das anschließend mit einem Methylierungsmittel wie Methylchlorid, Methylbromid, Methyljodid und Dimethylsulfat behandelt wird.

Eine mögliche Verfahrensvariante zur Herstellung der Benzylverbindungen III (Y = Stickstoff, L = Chlor, Brom) besteht darin, den aus der DE-A 36 23 921 bekannten 2-Methyl-phenylglyoxylsäuremethylester VII nach einer der vorstehend beschriebenen Methoden zuerst zu halogenieren und das Produkt in das entsprechende Oxim zu überführen:

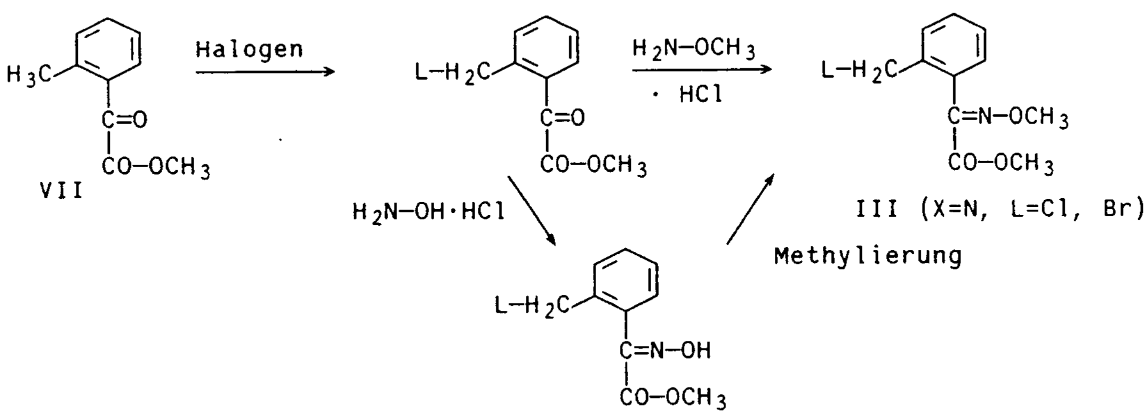


VII

III (X=N, L=Cl, Br)

Diejenigen ortho-substituierten Benzylverbindungen III, wobei die Abgangsgruppe L einen p-Toluolsulfonat-, p-Bromphenylsulfonat-, Methansulfonat- oder Trifluormethansulfonatrest bedeutet, sind aus den Benzylverbindungen III mit L = Chlor oder Brom, durch Umsetzung mit p-Toluolsulfonsäure, p-Bromphenylsulfonsäure, Methansulfonsäure oder Trifluormethansulfonsäure herstellbar.

Die Reaktion wird zweckmäßigerweise in einem Lösungs- bzw. Verdünnungsmittel, z.B. Dimethylformamid, in Gegenwart einer Base, z.B. Kaliumcarbonat, durchgeführt.

Die Reaktionstemperatur liegt im allgemeinen zwischen 20 und 130 °C.

Eine Verfahrensvariante besteht darin, die Benzylverbindungen III (L = Chlor, Brom) mit den Alkalimetallsalzen, bevorzugt den Natrium- oder Kaliumsalzen, der Sulfonsäuren in dem inerten Lösungsmittel umzusetzen.

Die ortho-substituierten Benzylester von Cyclopropancarbonsäuren I eignen sich als Fungizide und zur Bekämpfung von Schädlingen.

Die ortho-substituierten Benzylester von Cyclopropancarbonsäuren I zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an

den Samen dieser Pflanzen.

Speziell eignen sie sich zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen werden angewendet, indem man die Pilze oder die vor Pilzbefall zu schützenden Pflanzen, Saatgüter, Materialien oder den Erdboden mit einer fungizid wriksamen Menge der Wirkstoffe behandelt. Die Anwendung erfolgt vor oder nach der Infektion der Materialien, Pflanzen oder Samen durch die Pilze.

Die ortho-substituierten Benzylester von Cyclopropancarbonsäuren I eignen sich des weiteren zur Bekämpfung von Schädlingen aus der Klasse der Insekten, Spinnentiere und Nematoden. Sie können im Pflanzenschutz sowie auf dem Hygiene-, Vorratsschutz- und Veterinärsektor als Schädlingsbekämpfungsmitteleingesetzt werden.

Zu den schädlichen Insekten gehören aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grndiosella, Earias insulana, Elasmopalpus lignosellus, Eupoecilia ambiguella, Evetria bouliana, Feltia subterranea, Galleria mellonella, Grapholita funebrana, Grapholita molesta, Heliothis armigera, Heliothis virescens, Heliothis zea, Hellula undalis, Hibernia defoliaria, Hyphantria cunea, Hyponomeuta malinellus, Keifferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flamea, Pectinophora gossypiella, Peridroma saucia, Phalera bucephala, Phthorimaea operculella, Phyllocnistis citrella, Pieris brassicae, Plathypena scarbra, Plutella xylostella, Pseudoplusia includens, Phyacionia frustrana, Scrobipalpula absoluta, Sitotroga cerelella, Sparganothis pilleriana, Spodoptera frugiperda, Spodoptera littoralis, Spodoptera litura, Thaumatopoea pityocampa, Tortrix viridana, Trichoplusia ni, Zeiraphera canadensis.

Aus der Ordnung der Käfer (Coleoptera) beispielsweise Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Onlema oryzae, Ortiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus, Sitophilus granaria.

Aus der Ordnung der Zweiflügler (Diptera) beispielsweise Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossia morsitans, Haematobia

irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea, Tipula paludosa.

Aus der Ordnung der Thripse (Thysanoptera) beispielsweise Frankliniella fusca, Frankliniella occidentalis, Frankliniella tritici, Scirtothrips citri, Thrips oryzae, Thrips palmi, Thrips tabaci.

Aus der Ordnung der Hautflügler (Hymenoptera) beispielsweise Athalia rosae, Atta cephalotes, Atta sexdens, Atta texana, Hoplocampa minuta, Hoplocampa testudinea, Monomorium pharaonis, Solenopsis geminata, Solenopsis invicta.

Aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euchistus impictiventris, Leptoglossus phyllopus, Lygus lineolaris, Lygus pratensis, Nezara viridula, Piesma quadrata, Solubea insularis, Thyanta perditor.

Aus der Ordnung der Pflanzensauger (Homoptera) beispielsweise Acyrthosiphon onobrychis, Adelges laricis, Aphidula nasturtii, Aphis fabae, Aphis pomi, Aphis sambuci, Brachycaudus cardui, Brevicoryne brassicae, Cerosipha gossypii, Dreyfusia nordmannianae, Dreyfusia piceae, Dyasphis radicola, Dysaulacorthum pseudosolani, Empoasca fabae, Macrosiphum avenae, Macrosiphum euphorbiae, Macrosiphon rosae, Megoura viciae, Metopolophium dirhodum, Myzodes persicae, Myzus cerasi, Nilaparvata lugens, Pemphigus bursarius, Perkinsiella saccharicida, Phorodon humuli, Psylla mali, Psylla piri, Rhopalomyzus ascalonicus, Rhopalosiphum maidis, Sappaphis mala, Sappaphis mali, Schizaphis graminum, Schizoneura lanuginosa, Trialeurodes vaporariorum, Viteus vitifolii.

Aus der Ordnung der Termiten (Isoptera) beispielsweise Calotermes flavicollis, Leucotermes flavipes, Reticulitermes lucifugus, Termes natalensis.

Aus der Ordnung der Geradflügler (Orthoptera) beispielsweise Acheta domestica, Blatta orientalis, Blattella germanica, Forficula auricularia, Gryllotalpa gryllotalpa, Locusta migratoria, Melanoplus birittatus, Melanoplus femur-rubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus, Tachycines asynamorus.

Aus der Klasse der Arachnoidea beispielsweise Spinnentiere (Acarina) wie Amblyomma americanum, Amglyomma variegatum, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoni, Hyalomma truncatum, Ixodes ricinus, Ixodes rubicundus, Ornithodorus moubata, Otobins megnini, Paratetranychus pilosus, Permanyssus gallinae, Phyllocaptrata oleivora, Polyphagotarsonemus latus, Psoroptes ovis, Rhipicephalus appendiculatus, Rhipicephalus evertsi, Saccoptes scabiei, Tetranychus cinnabarinus, Tetranychus kanzawai, Tetranychus pacificus, Tetranychus telarius, Tetranychus urticae.

Aus der Klasse der Nematoden beispielsweise Wurzelgallennematoden, z.B. Meloidogyne hapla, Meloidogyne incognita, Meloidogyne javanica, Zysten bildende Nematoden, z.B. Globodera rostochiensis, Heterodera avenae, Heterodera glycinae, Heterodera schatii, Hetrodera triflolii, Stock- und Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Pratylenchus curvitatus, Pratylenchus goodeyi.

Die Wirkstoffe können in die üblichen Formulierungen übergeführt werden, wie Lösungen, Emulsionen, Suspensionen, Stäube, Pulver, Pasten und Granulate. Die Anwendungsformen richten sich nach den Verwendungszwecken; sie sollen in jedem Fall eine feine und gleichmäßige Verteilung des ortho-substituierten Benzylesters einer Cyclopropancarbonsäure gewährleisten. Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin-Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Die Formulierungen enthalten im allgemeinen zwischen 0,01 und 95 Gew.%, vorzugsweise zwischen 0,1 und 90 Gew.% des Wirkstoffs. Die Wirkstoffe werden dabei in einer Reinheit von 90 % bis 100 %, vorzugsweise 95 % bis 100 % (nach NMR-Spektrum) eingesetzt.

Beispiele für solche Zubereitungen sind:

I. eine Lösung aus 90 Gew.-Teilen der Verbindung Nr. 3 und 10 Gew.-Teilen N-Methyl-α-pyrrolidon, die zur Anwendung in Form kleinster Tropfen geeignet ist;

II. eine Mischung aus 20 Gew.-Teilen der Verbindung Nr. 4, 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure, 5 Gew.-Teilen des Anlagerungsproduktes von 40 Mol Ethylenoxid an 1 Mol Ricinusöl; durch feines Verteilen der Lösung in Wasser erhält man eine Dispersion.

III. eine wäßrige Dispersion aus 20 Gew.-Teilen der Verbindung Nr. 5, 40 Gew.-Teilen Cyclohexanon, 30 Gew.-Teilen Isobutanol, 20 Gew.-Teilen des Anlagerungsproduktes von 40 mol Ethylenoxid an 1 mol Ricinusöl;

IV. eine wäßrige Dispersion aus 20 Gew.-Teilen der Verbindung Nr. 6, 25 Gew.-Teilen Cyclohexanol, 65 Gew.-Teilen einer Mineralölfraktion vom Siedepunkt 210 bis 280 °C und 10 Gew.-Teilen des Anlagerungsproduktes von 40 mol Ethylenoxid an 1 mol Ricinusöl;

V. eine in einer Hammermühle vermahlene Mischung aus 80 Gew.-Teilen der Verbindung Nr. 7, 3 Gew.-Teilen des Natriumsalzes der Diisobutylnaphtalin-α-sulfonsäure, 10 Gew.-Teilen des Natriumsalzes einer Ligninsulfonsäure aus einer Sulfitablauge und 7 Gew.-Teilen pulverförmigem Kieselsäuregel; durch feines Verteilen der Mischung in Wasser erhält man eine Spritzbrühe;

VI. eine innige Mischung aus 3 Gew.-Teilen der Verbindung Nr. 15 und 97 Gew.-Teilen feinteiligem Kaolin; dieses Stäubemittel enthält 3 Gew.-% Wirkstoff;

VII. eine innige Mischung aus 30 Gew.-Teilen der Verbindung Nr. 103, 92 Gew.-Teilen pulverförmigem Kieselsäuregel und 8 Gew.-Teilen Paraffinöl, das auf die Oberfläche dieses Kieselsäuregels gesprüht wurde; diese Aufbereitung gibt dem Wirkstoff eine gute Haftfähigkeit;

VIII. eine stabile wäßrige Dispersion aus 40 Gew.-Teilen der Verbindung Nr. 109, 10 Gew.-Teilen des Natriumsalzes eines Phenolsulfonsäure-harnstoff-formaldehyd-Kondensates, 2 Gew.-Teilen Kieselgel und 48 Gew.-Teilen Wasser, die weiter verdünnt werden kann;

IX. eine stabile ölige Dispersion aus 20 Gew.-Teilen der Verbindung Nr. 177, 2 Gew.-Teilen des Calciumsalzes der Dodecylbenzolsulfonsäure, 8 Gew.-Teilen Fettalkohol-polyglykolether, 20 Gew.-Teilen des Natriumsalzes eines Phenolsulfonsäure-harnstoff-formaldehyd-Kondensates und 68 Gew.-Teilen eines paraffinischen Mineralöls.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Wirkstoffkonzentrationen in den anwendungsfertigen Zubereitungen können in größeren Bereichen variiert werden.

Ganz allgemein enthalten die Mittel zwischen 0,0001 und 95, vorzugsweise zwischen 0,01 und 90 Gew.-% Wirkstoff.

Formulierungen mit mehr als 95 Gew.-% Wirkstoff können mit gutem Erfolg im Ultra-Low-Volume-Verfahren (ULV) ausgebracht werden, wobei sogar der Wirkstoff ohne Zusätze verwendet werden kann.

Die Aufwandmengen in fungiziden Mitteln liegen je nach Art des gewünschten Effektes zwischen 0,02 und 3 kg Wirkstoff pro ha. Die neuen Verbindungen können auch im Materialschutz (Holzschutz) eingesetzt werden, z.B. gegen Paecilomyces variotii.

Bei der Saatgutbehandlung werden im allgemeinen Wirkstoffmengen von 0,001 bis 50 g, vorzugsweise 0,01 bis 10 g je Kilogramm Saatgut benötigt.

Die Aufwandmenge an Wirkstoff für die Bekämpfung von Insekten beträgt unter Freilandbedingungen 0,02 bis 10, vorzugsweise 0,1 bis 2,0 kg/ha.

Die erfindungsgemäßen Mittel können in diesen Anwendungsformen auch zusammen mit anderen Wirkstoffen vorliegen, z.B. mit Herbiziden, Insektiziden, Wachstumsregulatoren, Fungiziden oder auch mit Düngemitteln. Diese Mittel können zu den erfindungsgemäßen Mitteln im Gewichtsverhältnis 1 : 10 bis 10 : 1, gegebenenfalls auch erst unmittelbar vor der Anwendung (Tankmix), zugesetzt werden. Beim Vermischen mit Fungiziden oder Insektiziden erhält man dabei in vielen Fällen eine Vergrößerung des Wirkungsspektrums.

Die Mittel bzw. die daraus hergestellten gebrauchsfertigen Zubereitungen wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Herstellungsbeispiele

Beispiel 1 (Verbindung Nr. 3 in Tabelle 1)

Alpha-{2-[1-(n-propyl)-cyclopropylcarbonyloxymethyl]-phenyl}-beta-methoxyacrylsäuremethylester

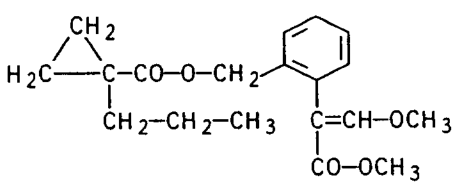

Eine Lösung aus 3,5 g (27 mmol) 1-n-Propyl-cyclopropancarbonsäure und 1,5 g (27 mmol) Kaliumhydroxid in 75 ml Ethanol wurde 2 Stunden bei 20 °C gerührt. Anschließend wurde das ausgefallene Kaliumsalz abgetrennt, mit 100 ml Diethylether gewaschen und in 50 ml Dimethylformamid suspendiert. Zu dieser Suspension gab man 5,7 g (20 mmol) Alpha-(2-brommethylphenyl)-beta-methoxy-acrylsäuremethylester und erhitzte dann 2 Stunden auf 90 °C. Nach Abkühlung auf 20 °C wurde das Gemisch mit 50 ml Wasser hydrolysiert. Dann wurde das Produkt mit Diethylether extrahiert und wie üblich isoliert. Die Reinigung erfolgte chromatographisch mit Kieselgel als Adsorbens und Cyclohexan als Laufmittel. Ausbeute: 66 %; Fp. : 62-64 °C.

Vorstufe 1a

1-Allyl-cyclopropancarbonsäure-tert.-butylester

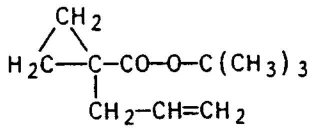

Zu einer Mischung aus 33,6 ml (0,24 mol) Diisopropylamin, 150 ml einer 1,5 molaren Lösung von n-Butyllithium in n-Hexan (≙ 0,24 mol Butyllithium) und 100 ml Tetrahydrofuran wurde bei (-70)°C eine Lösung von 34,1 g (0,24 mol) Cyclopropancarbonsäure-tert.-butylester in 25 ml Tetrahydrofuran getropft. Nach 3-stündigem Rühren bei (-70)°C wurde eine Lösung von 27,8 g (0,23 mol) Allylbromid in 25 ml Tetrahydrofuran zugetropft. Anschließend rührte man das Reaktionsgemisch noch 2 Stunden bei (-70)°C und dann 12 Stunden bei 20°C. Nach Hydrolyse mit 50 ml gesättigter wäßriger Ammoniumchloridlösung und nach der Phasentrennung wurde die organische Phase wie üblich auf das Produkt hin aufgearbeitet. Die Reinigung des Rohproduktes erfolgte destillativ. Ausbeute: 61 %; Sdp.: 86-88°C bei 30 mbar; farbloses Öl.

Vorstufe 1b

1-n-Propyl-cyclopropancarbonsäure-tert.-butylester

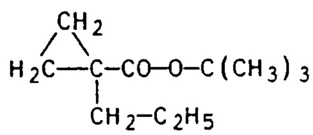

Bei 30°C und 10 bar Wasserstoffdruck wurden 25,5 g (0,14 mol) 1-Allyl-cyclopropancarbonsäure-tert.-butylester, gelöst in 150 ml Tetrahydrofuran, unter Zusatz von 6 g Aluminiumoxid, das 0,5 Gew.-% Palladium enthielt, hydriert. Nachdem kein Wasserstoff mehr aufgenommen wurde (Druckkonstanz), filtrierte man die Feststoffe ab und engte das Filtrat bis zur Trockene ein. Die Reinigung des Rohproduktes erfolgte destillativ. Ausbeute 87 %; Sdp.: 89°C bei 36 mbar; farbloses Öl.

Vorstufe 1c

1-n-Propyl-cyclopropancarbonsäure

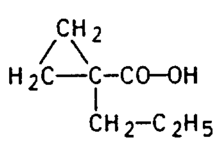

Eine Mischung aus 21,0 g (0,11 mol) 1-n-Propyl-cyclopropancarbonsäure-tert.-butylester und 13,0 g (0,11 mol) Trifluoressigsäure wurde 3 Stunden bei Rückflußtemperatur gerührt und dann in 20 ml verdünnte Natronlauge gegeben. Nach Extraktion von Nebenprodukten mit Diethylether wurde die wäßrige Phase mit verdünnter Salzsäure angesäuert und nochmals mit Diethylether extrahiert. Diese Etherphase wurde dann wie üblich auf das Produkt hin aufgearbeitet. Ausbeute: 95 %; farbloses Öl.

Beispiel 2 (Verbindung Nr. 4 in Tabelle 1)

2-[1-(n-propyl)-cyclopropylcarbonyloxymethyl]-phenyl-glyoxylsäuremethylester-O-methyloxim

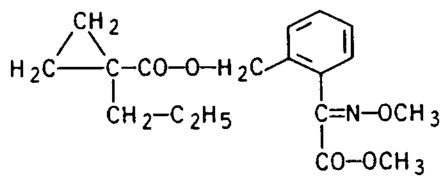

Eine Mischung aus 2,8 g (22 mmol) 1-n-Propyl-cyclopropancarbonsäure (hergestellt nach Beispiel 1c), 1,3 g (23 mmol) Kaliumhydroxid und 50 ml Ethanol wurde 1 Stunde bei 20 °C gerührt. Anschließend wurde das ausgefallene Kaliumsalz abgetrennt, mit 50 ml Diethylether gewaschen und in 100 ml Dimethylformamid suspendiert. Zu dieser Suspension gab man 4,3 g (15 mmol) 2-(Brommethyl)-phenyl-glyoxylsäuremethylester-O-methyloxim und erhitzte dann 2 Stunden auf 100 °C. Nach Abkühlung auf 20 °C wurde das Gemisch mit 50 ml Wasser hydrolysiert und analog Beispiel 1 auf das Produkt hin aufgearbeitet. Ausbeute: 38 %; Fp.: 56-59 °C; farblose Kristalle.

Beispiel 3 (Verbindung Nr. 5 in Tabelle 1)

Alpha-{2-[1-(allyl)-cyclopropylcarbonyloxymethyl]-phenyl}-beta-methoxy-acrylsäuremethylester

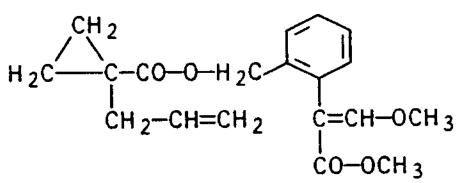

Eine Lösung aus 4,5 g (36 mmol) 1-Allyl-cyclopropancarbonsäure und 2,2 g (39 mmol) Kaliumhydroxid in 50 ml Ethanol wurde 2 Stunden bei 20 °C gerührt und dann eingeengt. Nach Überschichten mit Diethylether wurde der ausgefallene Niederschlag abgetrennt und mit Diethylether gewaschen. Die anschließende Umsetzung des Kaliumsalzes der 1-Allyl-cyclopropancarbonsäure in 50 ml N-Methylpyrrolidon mit 8,5 g (30 mmol) Alpha-(2-brommethylphenyl)-beta-methoxyacrylsäuremethylester sowie die Reinigung des Endproduktes erfolgte analog Beispiel 1. Ausbeute: 40 %; farbloses Öl.

Vorstufe 3a

1-Allyl-cyclopropancarbonsäure

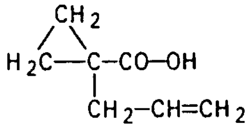

Analog Beispiel 1c wurden 24,0 g (0,13 mol) 1-Allyl-cyclopropancarbonsäure-tert.-butylester (hergestellt nach Beispiel 1a) mit 14,9 g (0,13 mol) Trifluoressigsäure umgesetzt. Ausbeute: 90 %; farbloses Öl.

Beispiel 4 (Verbindung Nr. 6 in Tabelle 1)

2-[1-(Allyl)-cyclopropylcarbonyloxymethyl]-phenyl-glyoxylsäuremethylester-O-methyloxim

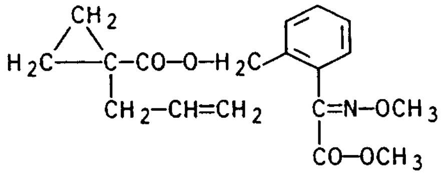

Analog Beispiel 3 wurden 2,3 g (18 mmol) 1-Allyl-cyclopropancarbonsäure (hergestellt nach Beispiel 1c) mit 1,1 g (20 mmol) Kaliumhydroxid in das Kaliumsalz der 1-Allyl-cyclopropancarbonsäure überführt und dieses wurde in 50 ml Dimethylformamid mit 4,3 g (15 mmol) 2-(Brommethyl)-phenyl-glyoxylsäuremethylester-O-methyloxim umgesetzt. Ausbeute: 84 %; farbloses Öl.

Beispiel 5 (Verbindung Nr. 7 in Tabelle 1)

Alpha-{2-[1-(trifluormethyl)-cyclopropylcarbonyloxymethyl]-phenyl}-beta-methoxyacrylsäuremethylester

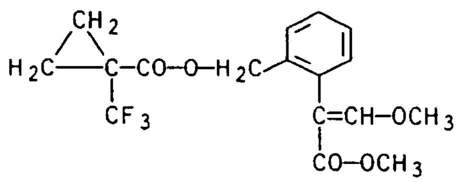

Eine Lösung aus 10,0 g (55 mmol) 1-Trifluormethyl-cyclopropancarbonsäure-ethylester und 3,4 g (61 mmol) Kaliumhydroxid in 150 ml Ethanol wurde 4 Stunden bei 40 °C gerührt und dann eingeengt. Nach Überschichten mit Diethylether wurde der ausgefallene Niederschlag abgetrennt, mit Diethylether gewaschen und in 100 ml Dimethylformamid suspendiert. Zu dieser Suspension gab man 10,0 g (35 mmol) Alpha-(2-Brommethylphenyl)-beta-methoxyacrylsäuremethylester und erhitzte dann 2 Stunden auf 95 °C. Nach Abkühlung auf 20 °C wurde das Gemisch mit 50 ml Wasser hydrolysiert. Dann wurde das Produkt mit Diethylether extrahiert und wie üblich isoliert.

Das ölige Rohprodukt wurde mit Pentan überschichtet und durch Reiben an der Gefäßwand zur Kristallisation gebracht. Ausbeute: 64 %; Fp.: 58-60 °C; weiße Kristalle.

Vorstufe 5a

Cyclopropan-1,1-dicarbonsäuremonoethylester

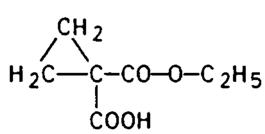

Eine Lösung aus 53 g (285 mmol) Cyclopropan-1,1-dicarbonsäurediethylester und 16,0 g (286 mmol) Kaliumhydroxid in 300 ml Ethanol wurde 3 Stunden bei 20 °C gerührt und dann zur Trockene eingeengt. Der Rückstand wurde in 100 ml Wasser gelöst, wonach Nebenprodukte mit 200 ml Methylenchlorid extrahiert wurden. Nachdem die wäßrige Phase mit verdünnter Salzsäure auf pH = 2 angesäuert wurde, extrahierte man das Produkt mit 200 ml Methyl-tert.-butylether und isolierte es wie üblich. Die Reinigung erfolgte destillativ. Ausbeute: 83 %; Sdp.: 99-102 °C bei 2,5 mbar; farbloses Öl.

Vorstufe 5b

1-Trifluormethyl-cyclopropancarbonsäure-ethylester

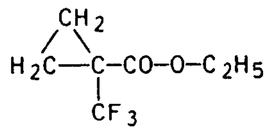

In einen 500 ml Rührautoklaven, der mit einer Legierung aus 70 % Nickel, 15 % Chrom und 15 % Molybdän ausgekleidet war, wurden bei (-70) ° C 31,6 g (0,20 mol) Cyclopropan-1,1-dicarbonsäuremonoethylester, 126 g (1,16 mol) Schwefeltetrafluorid, 100 ml Dichlormethan und 1,5 g (75 mmol) Fluorwasserstoff gegeben. Man erhitzte die Mischung 48 Stunden auf 80 ° C und vernichtete die gasförmigen Bestandteile, nach Abkühlung des Autoklaven auf 35 ° C, durch Einleiten in einen mit Kaliumhydroxid gefüllten Waschturm. Der Rückstand wurde in 100 ml Dichlormethan gelöst. Nach Waschen der Lösung mit 100 ml gesättigter Natriumhydrogencarbonat-Lösung wurde die organische Phase mit Natriumsulfat und Kaliumfluorid getrocknet und destillativ in die einzelnen Komponenten aufgetrennt. Ausbeute: 76 %; Sdp.: 141-142 ° C; Öl.

Beispiel 6 (Verbindung Nr. 8 in Tabelle 1)

Alpha-{2-[1-(trimethylsilyl)-cyclopropylcarbonyloxymethyl]-phenyl}-beta-methoxyacrylsäuremethylester

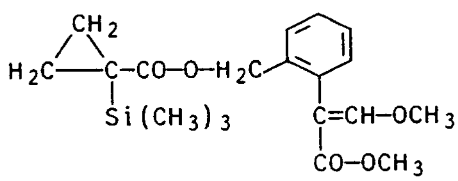

Analog Beispiel 3 wurden 4,5 g (28 mmol) 1-Trimethylsilylcyclopropancarbonsäure, gelöst in 80 ml Ethanol, mit 1,8 g (32 mmol) Kaliumhydroxid in das Kaliumsalz der Trimethylsilylcyclopropancarbonsäure überführt und diese wurde bei 100 ° C mit 5,7 g (20 mmol) Alpha-(2-brommethylphenyl)-beta-methoxy-acrylsäuremethylester umgesetzt. Ausbeute: 62 %; farbloses Öl.

Vorstufe 6a

1-Trimethylsilyl-cyclopropancarbonsäure

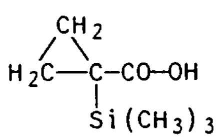

Zu einer Mischung aus 42,0 ml (0,30 mol) Diisopropylamin, 200 ml einer 1,5 molaren Lösung von n-Butyllithium in n-Hexan (≙ 0,30 mol Butyllithium) und 150 ml Tetrahydrofuran wurden bei (-78) ° C 12,9 g (0,15 mol) Cyclopropancarbonsäure und nach 30-minütigem Rühren bei (-78) ° C 81,3 g (0,75 mol) Trimethylchlorsilan zugetropft. Anschließend rührte man das Reaktionsgemisch noch 30 Minuten bei 0 ° C, versetzte es nach Erwärmen auf 20 ° C mit 150 ml Methanol und rührte weitere 30 Minuten. Nach der Hydrolyse mit Wasser wurde die organische Phase abgetrennt und eingeengt. Aus der aufkonzentrierten Lösung wurden farblose Kristalle erhalten. Ausbeute: 38 %; Fp.: 124-126 ° C.

Beispiel 7 (Verbindung Nr. 11 in Tabelle 1)

Alpha-{2-[1-(ethoxycarbonyl)-cyclopropylcarbonyloxymethyl]-phenyl}-beta-methoxyacrylsäuremethylester

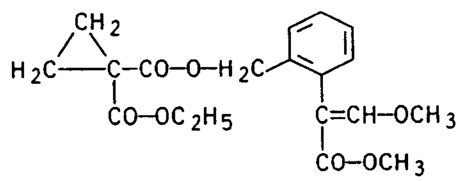

Analog Beispiel 1 wurden 7,9 g (50 mmol) Cyclopropan-1,1-dicarbonsäuremonoethylester (hergestellt nach Beispiel 5a) mit 2,8 g (50 mmol) Kaliumhydroxid in das Kaliumsalz des Cyclopropan-1,1-dicarbonsäuremonomethylesters überführt und dieses wurde anschließend mit 10 g (35 mmol) Alpha-[2-(brommethyl)-phenyl]-beta-methoxyacrylsäuremethylester umgesetzt. Die Reinigung des Produktes erfolgte destillativ. Ausbeute: 66 %; Sdp.: 220 °C bei 0,3 mbar; farbloses Öl.

Beispiel 8 (Verbindung Nr. 17 in Tabelle 1)

Alpha-{2-[1-(2,6-difluorphenyl)-cyclopropylcarbonyloxymethyl]-phenyl}-beta -methoxyacrylsäuremethylester

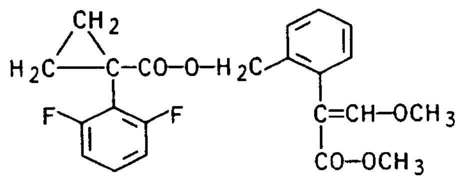

Analog Beispiel 1 wurden 12,8 g (65 mmol) 1-(2,6-Difluorphenyl)-cyclopropancarbonsäure, gelöst in 60 ml Ethanol, mit 4,0 g (71 mmol) Kaliumhydroxid in das Kaliumsalz der 2,6-Difluorphenyl-cyclopropancarbonsäure überführt und diese wurde anschließend bei 100 °C, in 100 ml N-Methylpyrrolidon, mit 11,4 g (40 mmol) Alpha-(2-brommethylphenyl)-beta-methoxyacrylsäure-methylester umgesetzt. Das erhaltene ölige Produkt wurde mit Diisopropylether überschichtet und durch Reiben an der Gefäßwand zur Kristallisation gebracht. Ausbeute: 86 %; Fp.: 108-110 °C; weiße Kristalle.

Vorstufe 8a

1-(2,6-Difluorphenyl)-cyclopropylnitril

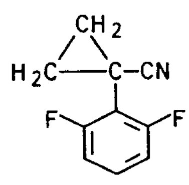

Zu einer Mischung aus 30,6 g (0,20 mol) 2,6-Difluorbenzylcyanid und 150,0 g (0,80 mol) 1,2-Dibromethan wurden 4,0 g Tetrabutylammoniumchlorid gegeben und anschließend 150 ml 50 %ige Natronlauge zugetropft. Nach 5 Stunden Rühren bei 60 °C hydrolysierte man mit Eiswasser. Aus dem erhaltenen Gemisch wurde das Produkt mit Diethylether extrahiert und in üblicher Weise isoliert. Die Reinigung erfolgte chromatographisch mit Kieselgel als Adsorbens und Cyclohexan als Laufmittel. Ausbeute: 52 %; Fp.: 56-58 °C; farblose Kristalle.

Vorstufe 8b

1-(2,6-Difluorphenyl)-cyclopropancarbonsäure

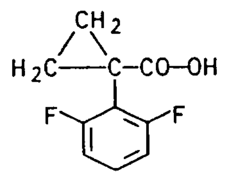

Eine Suspension aus 17,9 g (100 mmol) 1-(2,6-Difluorphenyl)-cyclopropylnitril in einem Gemisch aus 60 ml Eiswasser und 40 ml konzentrierter Schwefelsäure wurde 4 Stunden auf Rückflußtemperatur erhitzt. Nach dem Abkühlen auf 25 ° C wurde der entstandene Niederschlag abgetrennt, mit Wasser gewaschen und getrocknet. Ausbeute: 89 %; Fp.: 150-153 ° C; farblose Kristalle.

Beispiel 9 (Verbindung Nr. 18 in Tabelle 1)

2-[1-(2,6-Difluorphenyl)-cyclopropylcarbonyloxymethyl]-phenylglyoxylsäuremethylester-O-methyloxim

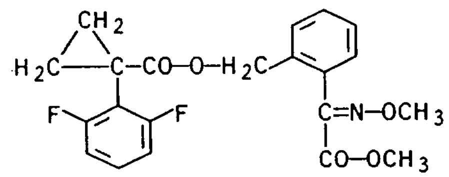

Analog Beispiel 2 wurden 11,8 g (60 mmol) 1-(2,6-Difluorphenyl)-cyclopropancarbonsäure (hergestellt nach Beispiel 8b), gelöst in 60 ml Ethanol, mit 3,6 g (64 mmol) Kaliumhydroxid in das Kaliumsalz der 2,6-Difluorphenyl-cyclopropancarbonsäure überführt, und diese wurde anschließend bei 90 ° C, in 100 ml N-Methylpyrrolidon, mit 13,3 g (47 mmol) 2-(Brommethyl)-phenyl-glyoxylsäuremethylester-O-methyloxim umgesetzt. Das erhaltene ölige Produkt wurde mit Diisopropylether überschichtet und durch Reiben an der Gefäßwand zur Kristallisation gebracht. Ausbeute: 85 %; Fp.: 122-123 ° C; weiße Kristalle.

Beispiel 10 (Verbindung Nr. 29 in Tabelle 1)

Alpha-{2-[1-(4-fluorbenzyl)-cyclopropylcarbonyloxymethyl]-phenyl}-beta-methoxyacrylsäuremethylester

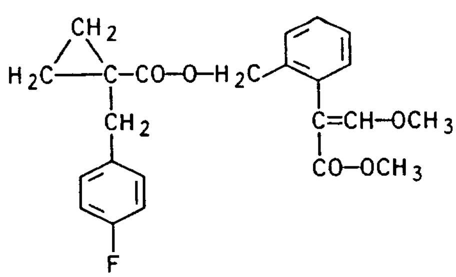

Analog Beispiel 3 wurden 6,3 g (32 mmol) 1-(4-Fluorbenzyl)-cyclopropancarbonsäure mit 2,0 g (36 mmol) Kaliumhydroxid in das Kaliumsalz der Fluorbenzyl-cyclopropancarbonsäure überführt und dieses wurde anschließend bei 100 ° C mit 6,3 g (22 mmol) Alpha-(2-brommethylphenyl)-beta-methoxyacrylsäuremethylester umgesetzt. Ausbeute: 54 %; farbloses Öl.

Vorstufe 10a

1-(4-Fluorbenzyl)-cyclopropancarbonsäure-tert.-butylester

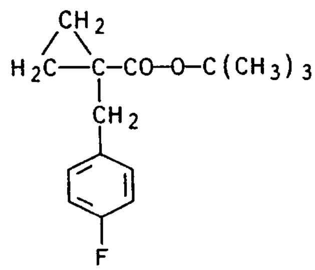

Analog Vorstufe 1a wurden 34,1 g (0,24 mol) Cyclopropancarbonsäure-tert.-butylester in einen Lithiumkomplex überführt und dieser wurde mit 30,2 g (0,16 mol) 4-Fluorbenzylbromid umgesetzt. Die Reinigung erfolgte chromatographisch mit Kieselgel als Adsorbens und Cyclohexan als Laufmittel. Ausbeute: 72 %; farbloses Öl.

Vorstufe 10b

1-(4-Fluorbenzyl)-cyclopropancarbonsäure

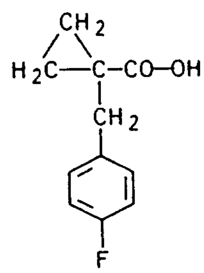

Analog Vorstufe 1c wurden 25,0 g (0,10 mol) 1-(4-Fluorbenzyl)-cyclopropancarbonsäure-tert.-butylester (hergestellt nach Vorstufe 10a) mit 14,3 g (0,13 mol) Trifluoressigsäure umgesetzt. Ausbeute: 90 %; farbloses Öl.

Beispiel 11 (Verbindung Nr. 30 in Tabelle 1)

2-[1-(4-Fluorbenzyl)-cyclopropylcarbonyloxymethyl]-phenyl-glyoxylsäuremethylester-O-methyloxim

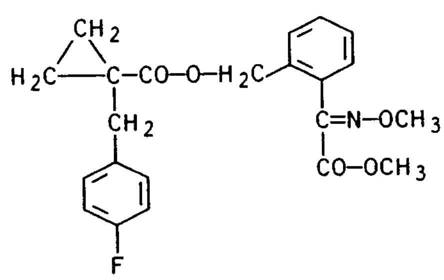

Analog Beispiel 2 wurden 5,8 g (30 mmol) 1-(4-Fluorbenzyl)-cyclopropancarbonsäure (hergestellt nach Vorstufe 10a) mit 1,8 g (32 mmol) Kaliumhydroxid in das Kaliumsalz der Fluorbenzyl-cyclopropancarbonsäure überführt und dieses wurde anschließend mit 6,3 g (22 mmol) 2-(Brommethyl)-phenylglyoxylsäuremethylester-O-methyloxim umgesetzt. Die Reinigung erfolgte chromatographisch mit Kieselgel als Adsorbens und Toluol als Laufmittel. Ausbeute: 62 %; farbloses Öl.

Die physikalischen Daten der Endprodukte I sind den folgenden Tabellen 1 bis 4 zu entnehmen, in denen noch weitere Verbindungen I aufgeführt sind, welche auf die gleichen Weisen hergestellt wurden oder herstellbar sind.

Tabelle 1

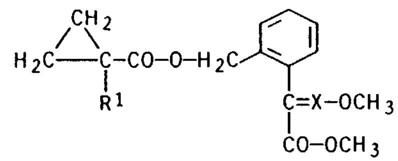
Ia

| Nr. | $R^1$ | X | Konfig.*) | physik. Daten (NMR in $CDCl_3$ [ppm]; TMS als Standard) |
|---|---|---|---|---|
| 1 | $H_3C-CH_2-$ | CH | | |
| 2 | $H_3C-CH_2-$ | N | | |
| 3 | $H_3C-CH_2-CH_2-$ | CH | E | 62-64°C; NMR: 0.68(m,2H); 0.89(t,3H); 1.20(m,2H); 1.51(m,4H); 3.68(s,3H); 3.79(s,3H); 5.00(s,2H); 7.13-7.41(m,4H); 7.57(s,1H). |
| 4 | $H_3C-CH_2-CH_2-$ | N | E | 56-59°C; NMR: 0.68(m,2H); 0.87(t,3H); 1.17(m,2H); 1.47(m,4H); 3.87(s,3H); 4.05(s,3H); 4.95(s,2H); 7.13-7.45(m,4H). |
| 5 | $H_2C=CH-CH_2-$ | CH | E | Öl; NMR: 0.76(m,2H); 1.24(m,2H); 2.35(d,2H); 2.69(s,3H); 3.80(s,3H); 5.00(s,2H); 5.04(d,2H); 5.85(m,1H); 7.13-7.43(m,4H); 7.59(s,1H). |
| 6 | $H_2C=CH-CH_2-$ | N | E | Öl; NMR: 0.73(m,2H); 1.20(m,2H); 2.32(d,2H); 3.88(s,3H); 4.03(s,3H); 4.98(s,2H); 5.05(d,2H); 5.83-m,1H); 7.15-7.47(m,4H). |
| 7 | $CF_3-$ | CH | E | 58-60°C; NMR: 1.27(m,2H); 1.40(m,2H); 3.63(s,3H); 3.70(s,3H); 5.10(s,2H); 7.10-7.40(m,4H); 7.53(s,1H). |

*) Stellung der Substituenten an der Doppelbindung $-C(COOCH_3)=X-OCH_3$

| Nr. | $R^1$ | X | Konfig.*) | physik. Daten (NMR in $CDCl_3$ [ppm]; TMS als Standard) |
|---|---|---|---|---|
| 8 | $(CH_3)_3Si-$ | CH | E | Öl; NMR: 0.03(s,9H); 0.76(m,2H); 1.19(m,2H); 3.70(s,3H); 3.80(s,3H); 4.99(s,2H); 7.13-7.44(m,4H); 7.57(s,1H). |
| 9 | CN- | CH | E | 91-92°C; NMR: 1.60(m,2H); 1.67(m,2H); 3.70(s,3H); 3.84(s,3H); 7.12-7.50(m,4H); 7.60(s,1H). |
| 10 | CN- | N | | |
| 13 | $2-Br-C_6H_4-$ | CH | E | 122-123°C; NMR: 1.22(m,2H); 1.75(m,2H); 3.67(s,3H); 3.78(s,3H); 5.00(s,2H); 7.07-7.60(m,8H); 7.53(s,1H). |
| 14 | $2-Br-C_6H_4-$ | N | E | 106-107°C; NMR: 1.20(m,2H); 1.76(m,2H); 3.83(s,3H); 4.00(s,3H); 4.96(s,2H); 7.10-7.60(m,8H). |
| 15 | $2,4-F_2-C_6H_3-$ | CH | E | 83-84°C; NMR: 1.19(m,2H); 1.65(m,2H); 3.70(s,3H); 3.82(s,3H); 5.00(s,2H); 6.77-7.31(m,7H); 7.57(s,1H). |
| 16 | $2,4-F_2-C_6H_3-$ | N | | |
| 17 | $2,6-F_2-C_6H_3-$ | CH | E | 108-110°C; NMR: 1.25(m,2H); 1.77(m,2H); 3.67(s,3H); 3.80(s,3H); 5.00(s,2H); 6.84-7.28(m,7H); 7.56(s,1H). |
| 18 | $2,6-F_2-C_6H_3-$ | N | E | 122-123°C; NMR: 1.27(m,2H); 1.73(m,2H); 3.87(s,3H); 4.03(s,3H); 4.97(s,2H); 6.82-7.37(m,7H). |
| 19 | $2-F-6-Cl-C_6H_3-$ | CH | E | 126-127°C; NMR: 1.28(m,2H); 1.80(m,2H); 3.67(s,3H); 3.80(s,3H); 5.00(s,2H); 7.92-7.25(m,7H); 7.53(s,1H). |
| 20 | $2-F-6-Cl-C_6H_3-$ | N | E | 89-90°C |

*) Stellung der Substituenten an der Doppelbindung $-C(COOCH_3)=X-OCH_3$

| Nr. | $R^1$ | X | Konfig.*) | physik. Daten (NMR in $CDCl_3$ [ppm]; TMS als Standard) |
|---|---|---|---|---|
| 22 | 2,3,6-$Cl_3$-$C_6H_2$- | N | | |
| 23 | $C_6H_5$-$CH_2$- | CH | E | öl; NMR: 0.78(m,2H); 1.27(m,2H); 2.98(s,2H); 3.63(s,3H); 3.70(s,3H); 4.79(s,2H); 7.08-7.27(m,9H); 7.52(s,1H). |
| 24 | $C_6H_5$-$CH_2$- | N | E | öl; NMR: 0.77(m,2H); 1.23(m,2H); 2.98(s,2H); 3.83(s,3H); 4.00(s,3H); 4.93(s,2H); 7.12-7.37(m,9H). |
| 25 | 2-F-$C_6H_4$-$CH_2$ | CH | | |
| 26 | 2-F-$C_6H_4$-$CH_2$- | N | | |
| 27 | 3-F-$C_6H_4$-$CH_2$- | CH | E | öl |
| 28 | 3-F-$C_6H_4$-$CH_2$- | N | E | öl |
| 29 | 4-F-$C_6H_4$-$CH_2$- | CH | E | öl; NMR: 0.80(m,2H); 1.38(m,2H); 2.94(s,2H); 3.68(s,3H); 3.74(s,3H); 4.98(s,2H); 6.89-7.30(m,8H); 7.53(s,1H). |
| 30 | 4-F-$C_6H_4$-$CH_2$- | N | E | öl; NMR: 0.75(m,2H); 1.25(m,2H); 2.93(s,2H); 3.83(s,3H); 4.00(s,3H); 4.92(s,2H); 6.83-6.37(m,8H). |
| 31 | 2-Cl-$C_6H_4$-$CH_2$- | CH | | |
| 32 | 2-Cl-$C_6H_4$-$CH_2$ | N | | |
| 33 | 3-Cl-$C_6H_4$-$CH_2$- | CH | E | öl |
| 34 | 3-Cl-$C_6H_4$-$CH_2$- | N | E | öl |
| 35 | 4-Cl-$C_6H_4$-$CH_2$- | CH | E | öl |
| 36 | 4-Cl-$C_6H_4$-$CH_2$- | N | E | öl |
| 37 | 2-Br-$C_6H_4$-$CH_2$- | CH | | |
| 38 | 2-Br-$C_6H_4$-$CH_2$- | N | | |
| 39 | 3-Br-$C_6H_4$-$CH_2$- | CH | | |
| 40 | 3-Br-$C_6H_4$-$CH_2$- | N | | |
| 41 | 4-Br-$C_6H_4$-$CH_2$- | CH | E | öl |

*) Stellung der Substituenten an der Doppelbindung -C($COOCH_3$)=X-$OCH_3$

| Nr. | $R^1$ | X | Konfig.*) | physik. Daten (NMR in $CDCl_3$ [ppm]; TMS als Standard) |
|---|---|---|---|---|
| 42 | $4-Br-C_6H_4-CH_2-$ | N | E | öl |
| 43 | $2-CH_3-C_6H_4-CH_2-$ | CH | | |
| 44 | $2-CH_3-C_6H_4-CH_2-$ | N | | |
| 45 | $3-CH_3-C_6H_4-CH_2-$ | CH | E | öl |
| 46 | $3-CH_3-C_6H_4-CH_2-$ | N | E | öl |
| 47 | $4-CH_3-C_6H_4-CH_2-$ | CH | | |
| 48 | $4-CH_3-C_6H_4-CH_2-$ | N | | |
| 49 | $2-CH_3O-C_6H_4-CH_2-$ | CH | | |
| 50 | $2-CH_3O-C_6H_4-CH_2-$ | N | | |
| 51 | $3-CH_3O-C_6H_4-CH_2-$ | CH | | |
| 52 | $3-CH_3O-C_6H_4-CH_2-$ | N | | |
| 53 | $4-CH_3O-C_6H_4-CH_2-$ | CH | | |
| 54 | $4-CH_3O-C_6H_4-CH_2-$ | N | | |
| 55 | $2-C_2H_5O-C_6H_4-CH_2-$ | CH | | |
| 56 | $2-C_2H_5O-C_6H_4-CH_2-$ | N | | |
| 57 | $3-C_2H_5O-C_6H_4-CH_2-$ | CH | | |
| 58 | $3-C_2H_5O-C_6H_4-CH_2-$ | N | | |
| 59 | $4-C_2H_5O-C_6H_4-CH_2-$ | CH | | |
| 60 | $4-C_2H_5O-C_6H_4-CH_2-$ | N | | |
| 61 | $4-(CH_3)_3C-C_6H_4-CH_2-$ | CH | E | öl |
| 62 | $4-(CH_3)_3C-C_6H_4-CH_2-$ | N | E | öl |
| 63 | $2-CF_3-C_6H_4-CH_2-$ | CH | | |
| 64 | $2-CF_3-C_6H_4-CH_2-$ | N | | |
| 65 | $3-CF_3-C_6H_4-CH_2-$ | CH | | |
| 66 | $3-CF_3-C_6H_4-CH_2-$ | N | | |

*) Stellung der Substituenten an der Doppelbindung $-C(COOCH_3)=X-OCH_3$

| Nr. | $R^1$ | X | Konfig.*) | physik. Daten (NMR in $CDCl_3$ [ppm]; TMS als Standard) |
|---|---|---|---|---|
| 67 | $4-CF_3-C_6H_4-CH_2-$ | CH | | |
| 68 | $4-CF_3-C_6H_4-CH_2-$ | N | | |
| 69 | $2,4-F_2-C_6H_3-CH_2-$ | CH | | |
| 70 | $2,4-F_2-C_6H_3-CH_2-$ | N | | |
| 71 | $2,6-F_2-C_6H_3-CH_2-$ | CH | | |
| 72 | $2,6-F_2-C_6H_3-CH_2-$ | N | | |
| 73 | $2,4-Cl_2-C_6H_3-CH_2-$ | CH | | |
| 74 | $2,4-Cl_2-C_6H_3-CH_2-$ | N | | |
| 75 | $2,6-Cl_2-C_6H_3-CH_2-$ | CH | | |
| 76 | $2,6-Cl_2-C_6H_3-CH_2-$ | N | | |
| 77 | $2-F-6-Cl-C_6H_3-CH_2-$ | CH | | |
| 78 | $2-F-6-Cl-C_6H_3-CH_2-$ | N | | |
| 79 | $3,4-Cl_2-C_6H_3-CH_2-$ | CH | | |
| 80 | $3,4-Cl_2-C_6H_3-CH_2-$ | N | | |
| 81 | $2,3,6-Cl_3-C_6H_2-CH_2-$ | CH | | |
| 82 | $2,3,6-Cl_3-C_6H_2-CH_2-$ | N | | |
| 83 | $2,4-(CH_3)_2-C_6H_3-CH_2-$ | CH | | |
| 84 | $2,4-(CH_3)_2-C_6H_3-CH_2-$ | N | | |
| 85 | $2,6-(CH_3)_2-C_6H_3-CH_2-$ | CH | | |
| 86 | $2,6-(CH_3)_2-C_6H_3-CH_2-$ | N | | |
| 87 | $3,4-(CH_3)_2-C_6H_3-CH_2-$ | CH | | |
| 88 | $3,4-(CH_3)_2-C_6H_3-CH_2-$ | N | | |
| 89 | $2,4-(CH_3O)_2-C_6H_3-CH_2-$ | CH | | |
| 90 | $2,4-(CH_3O)_2-C_6H_3-CH_2-$ | N | | |
| 91 | $2,6-(CH_3O)_2-C_6H_3-CH_2-$ | CH | | |

*) Stellung der Substituenten an der Doppelbindung $-C(COOCH_3)=X-OCH_3$

| Nr. | $R^1$ | X | Konfig.*) | physik. Daten (NMR in $CDCl_3$ [ppm]; TMS als Standard) |
|---|---|---|---|---|
| 92 | 2,6-$(CH_3O)_2$-$C_6H_3$-$CH_2$- | N | | |
| 93 | 3,4-$(CH_3O)_2$-$C_6H_3$-$CH_2$- | CH | | |
| 94 | 3,4-$(CH_3O)_2$-$C_6H_3$-$CH_2$- | N | | |
| 95 | 3,4-$(C_2H_5O)_2$-$C_6H_3$-$CH_2$- | CH | | |
| 96 | 3,4-$(C_2H_5O)_2$-$C_6H_3$-$CH_2$- | N | | |
| 97 | $C_6H_5$-$CH_2$-$CH_2$-$CH_2$- | CH | | |
| 98 | $C_6H_5$-$CH_2$-$CH_2$-$CH_2$- | N | | |
| 99 | 2-F-$C_6H_4$-$CH_2$-$CH_2$-$CH_2$- | CH | | |
| 100 | 2-F-$C_6H_4$-$CH_2$-$CH_2$-$CH_2$- | N | | |
| 101 | 3-F-$C_6H_4$-$CH_2$-$CH_2$-$CH_2$- | CH | | |
| 102 | 3-F-$C_6H_4$-$CH_2$-$CH_2$-$CH_2$- | N | | |
| 103 | 4-F-$C_6H_4$-$CH_2$-$CH_2$-$CH_2$- | CH | E | Öl; NMR: 0.67(m,2H); 1.20(m,2H); 1.53(m,2H); 1.75(m,2H); 2.55(m,2H); 3.67(s,3H); 3.80(s,3H); 5.00(s,2H); 6.90-7.38(m,8H); 7.58(s,1H). |
| 104 | 4-F-$C_6H_4$-$CH_2$-$CH_2$-$CH_2$- | N | E | Öl; NMR: 0.67(m,2H); 1.18(m,2H); 1.53(m,2H); 1.73(m,2H); 2.55(m,2H); 3.85(s,3H); 4.02(s,3H); 4.95(s,2H); 6.88-7.40(m,8H). |
| 105 | 2-Cl-$C_6H_4$-$CH_2$-$CH_2$-$CH_2$- | CH | | |
| 106 | 2-Cl-$C_6H_4$-$CH_2$-$CH_2$-$CH_2$- | N | | |
| 107 | 3-Cl-$C_6H_4$-$CH_2$-$CH_2$-$CH_2$- | CH | | |
| 108 | 3-Cl-$C_6H_4$-$CH_2$-$CH_2$-$CH_2$- | N | | |
| 109 | 4-Cl-$C_6H_4$-$CH_2$-$CH_2$-$CH_2$- | CH | E | Öl |
| 110 | 4-Cl-$C_6H_4$-$CH_2$-$CH_2$-$CH_2$- | N | E | Öl |
| 111 | 2-Br-$C_6H_4$-$CH_2$-$CH_2$-$CH_2$- | CH | | |
| 112 | 2-Br-$C_6H_4$-$CH_2$-$CH_2$-$CH_2$- | N | | |

*) Stellung der Substituenten an der Doppelbindung -C($COOCH_3$)=X-$OCH_3$

| Nr. | $R^1$ | X | Konfig.*) | physik. Daten (NMR in $CDCl_3$ [ppm]; TMS als Standard) |
|---|---|---|---|---|
| 113 | 3-Br-$C_6H_4$-$CH_2$-$CH_2$-$CH_2$- | CH | | |
| 114 | 3-Br-$C_6H_4$-$CH_2$-$CH_2$-$CH_2$- | N | | |
| 115 | 4-Br-$C_6H_4$-$CH_2$-$CH_2$-$CH_2$- | CH | | |
| 116 | 4-Br-$C_6H_4$-$CH_2$-$CH_2$-$CH_2$- | N | | |
| 117 | 2-$CH_3$-$C_6H_4$-$CH_2$-$CH_2$-$CH_2$- | CH | | |
| 118 | 2-$CH_3$-$C_6H_4$-$CH_2$-$CH_2$-$CH_2$- | N | | |
| 119 | 3-$CH_3$-$C_6H_4$-$CH_2$-$CH_2$-$CH_2$- | CH | | |
| 120 | 3-$CH_3$-$C_6H_4$-$CH_2$-$CH_2$-$CH_2$- | N | | |
| 121 | 4-$CH_3$-$C_6H_4$-$CH_2$-$CH_2$-$CH_2$- | CH | | |
| 122 | 4-$CH_3$-$C_6H_4$-$CH_2$-$CH_2$-$CH_2$- | N | | |
| 123 | 2-$CH_3O$-$C_6H_4$-$CH_2$-$CH_2$-$CH_2$- | CH | | |
| 124 | 2-$CH_3O$-$C_6H_4$-$CH_2$-$CH_2$-$CH_2$- | N | | |
| 125 | 3-$CH_3O$-$C_6H_4$-$CH_2$-$CH_2$-$CH_2$- | CH | | |
| 126 | 3-$CH_3O$-$C_6H_4$-$CH_2$-$CH_2$-$CH_2$- | N | | |
| 127 | 4-$CH_3O$-$C_6H_4$-$CH_2$-$CH_2$-$CH_2$- | CH | | |
| 128 | 4-$CH_3O$-$C_6H_4$-$CH_2$-$CH_2$-$CH_2$- | N | | |
| 129 | 2-$C_2H_5O$-$C_6H_4$-$CH_2$-$CH_2$-$CH_2$- | CH | | |
| 130 | 2-$C_2H_5O$-$C_6H_4$-$CH_2$-$CH_2$-$CH_2$- | N | | |
| 131 | 3-$C_2H_5O$-$C_6H_4$-$CH_2$-$CH_2$-$CH_2$- | CH | | |
| 132 | 3-$C_2H_5O$-$C_6H_4$-$CH_2$-$CH_2$-$CH_2$- | N | | |

*) Stellung der Substituenten an der Doppelbindung $-C(COOCH_3)=X-OCH_3$

| Nr. | $R^1$ | X | Konfig.*) | physik. Daten (NMR in $CDCl_3$ [ppm]; TMS als Standard) |
|---|---|---|---|---|
| 133 | $4-C_2H_5O-C_6H_4-CH_2-CH_2-CH_2-$ | CH | | |
| 134 | $4-C_2H_5O-C_6H_4-CH_2-CH_2-CH_2-$ | N | | |
| 135 | $4-(CH_3)_3C-C_6H_4-CH_2-CH_2-CH_2-$ | CH | | |
| 136 | $4-(CH_3)_3C-C_6H_4-CH_2-CH_2-CH_2-$ | N | | |
| 137 | $2-CF_3-C_6H_4-CH_2-CH_2-CH_2-$ | CH | | |
| 138 | $2-CF_3-C_6H_4-CH_2-CH_2-CH_2-$ | N | | |
| 139 | $3-CF_3-C_6H_4-CH_2-CH_2-CH_2-$ | CH | | |
| 140 | $3-CF_3-C_6H_4-CH_2-CH_2-CH_2-$ | N | | |
| 141 | $4-CF_3-C_6H_4-CH_2-CH_2-CH_2-$ | CH | | |
| 142 | $4-CF_3-C_6H_4-CH_2-CH_2-CH_2-$ | N | | |
| 143 | $2,4-F_2-C_6H_3-CH_2-CH_2-CH_2-$ | CH | | |
| 144 | $2,4-F_2-C_6H_3-CH_2-CH_2-CH_2-$ | N | | |
| 145 | $2,6-F_2-C_6H_3-CH_2-CH_2-CH_2-$ | CH | | |
| 146 | $2,6-F_2-C_6H_3-CH_2-CH_2-CH_2-$ | N | | |
| 147 | $2,4-Cl_2-C_6H_3-CH_2-CH_2-CH_2-$ | CH | | |

*) Stellung der Substituenten an der Doppelbindung $-C(COOCH_3)=X-OCH_3$

| Nr. | $R^1$ | X | Konfig.*) | physik. Daten (NMR in $CDCl_3$ [ppm]; TMS als Standard) |
|---|---|---|---|---|
| 148 | $2,4-Cl_2-C_6H_3-CH_2-CH_2-CH_2-$ | N | | |
| 149 | $2,6-Cl_2-C_6H_3-CH_2-CH_2-CH_2-$ | CH | | |
| 150 | $2,6-Cl_2-C_6H_3-CH_2-CH_2-CH_2-$ | N | | |
| 151 | $2-F-6-Cl-C_6H_3-CH_2-CH_2-CH_2-$ | CH | | |
| 152 | $2-F-6-Cl-C_6H_3-CH_2-CH_2-CH_2-$ | N | | |
| 153 | $3,4-Cl_2-C_6H_3-CH_2-CH_2-CH_2-$ | CH | | |
| 154 | $3,4-Cl_2-C_6H_3-CH_2-CH_2-CH_2-$ | N | | |
| 155 | $2,3,6-Cl_3-C_6H_2-CH_2-CH_2-CH_2-$ | CH | | |
| 156 | $2,3,6-Cl_3-C_6H_2-CH_2-CH_2-CH_2-$ | N | | |
| 157 | $2,4-(CH_3)_2-C_6H_3-CH_2-CH_2-CH_2-$ | CH | | |
| 158 | $2,4-(CH_3)_2-C_6H_3-CH_2-CH_2-CH_2-$ | N | | |
| 159 | $2,6-(CH_3)_2-C_6H_3-CH_2-CH_2-CH_2-$ | CH | | |

*) Stellung der Substituenten an der Doppelbindung $-C(COOCH_3)=X-OCH_3$

| Nr. | $R^1$ | X | Konfig.*) | physik. Daten (NMR in $CDCl_3$ [ppm]; TMS als Standard) |
|---|---|---|---|---|
| 160 | $2,6-(CH_3)_2-C_6H_3-CH_2-CH_2-CH_2-$ | N | | |
| 161 | $3,4-(CH_3)_2-C_6H_3-CH_2-CH_2-CH_2-$ | CH | | |
| 162 | $3,4-(CH_3)_2-C_6H_3-CH_2-CH_2-CH_2-$ | N | | |
| 163 | $2,4-(CH_3O)_2-C_6H_3-CH_2-CH_2-CH_2-$ | CH | | |
| 164 | $2,4-(CH_3O)_2-C_6H_3-CH_2-CH_2-CH_2-$ | N | | |
| 165 | $2,6-(CH_3O)_2-C_6H_3-CH_2-CH_2-CH_2-$ | CH | | |
| 166 | $2,6-(CH_3O)_2-C_6H_3-CH_2-CH_2-CH_2-$ | N | | |
| 167 | $3,4-(CH_3O)_2-C_6H_3-CH_2-CH_2-CH_2-$ | CH | | |
| 168 | $3,4-(CH_3O)_2-C_6H_3-CH_2-CH_2-CH_2-$ | N | | |
| 169 | $3,4-(C_2H_5O)_2-C_6H_3-CH_2-CH_2-CH_2-$ | CH | | |
| 170 | $3,4-(C_2H_5O)_2-C_6H_3-CH_2-CH_2-CH_2-$ | N | | |
| 171 | $C_6H_5-CH=CH-CH_2-$ | CH | | |
| 172 | $C_6H_5-CH=CH-CH_2-$ | N | | |
| 173 | $2-F-C_6H_4-CH=CH-CH_2-$ | CH | | |

*) Stellung der Substituenten an der Doppelbindung $-C(COOCH_3)=X-OCH_3$

| Nr. | R1 | X | Konfig.*) | physik. Daten (NMR in $CDCl_3$ [ppm]; TMS als Standard) |
|---|---|---|---|---|
| 174 | $2\text{-F-}C_6H_4\text{-CH=CH-CH}_2\text{-}$ | N | | |
| 175 | $3\text{-F-}C_6H_4\text{-CH=CH-CH}_2\text{-}$ | CH | | |
| 176 | $3\text{-F-}C_6H_4\text{-CH=CH-CH}_2\text{-}$ | N | | |
| 177 | $E\text{-4-F-}C_6H_4\text{-CH=CH}_2\text{-CH}_2\text{-}$ | CH | E | Öl; NMR: 0.77(m,2H); 1.23(m,2H); 2.45(d,2H); 3.67(s,3H); 3.77(s,3H); 5.00(s,2H); 6.18(m,1H); 6.33(d,1H); 6.92-7.42(m,8H); 7.58(s,1H). |
| 178 | $E\text{-4-F-}C_6H_4\text{-CH=CH}_2\text{-CH}_2\text{-}$ | N | E | Öl; NMR: 0.78(m,2H); 1.23(m,2H); 2.43(d,2H); 3.83(s,3H); 4.03(s,3H); 5.00(s,2H); 6.13(m,1H); 6.33(d,1H); 6.92-7.40(m,8H). |
| 179 | $2\text{-Cl-}C_6H_4\text{-CH=CH-CH}_2\text{-}$ | CH | | |
| 180 | $2\text{-Cl-}C_6H_4\text{-CH=CH-CH}_2\text{-}$ | N | | |
| 181 | $3\text{-Cl-}C_6H_4\text{-CH=CH-CH}_2\text{-}$ | CH | | |
| 182 | $3\text{-Cl-}C_6H_4\text{-CH=CH-CH}_2\text{-}$ | N | | |
| 183 | $E\text{-4-Cl-}C_6H_4\text{-CH=CH}_2\text{-CH}_2\text{-}$ | CH | E | Öl, |
| 184 | $E\text{-4-Cl-}C_6H_4\text{-CH=CH}_2\text{-CH}_2\text{-}$ | N | E | Öl, |
| 185 | $2\text{-Br-}C_6H_4\text{-CH=CH-CH}_2\text{-}$ | CH | | |
| 186 | $2\text{-Br-}C_6H_4\text{-CH=CH-CH}_2\text{-}$ | N | | |
| 187 | $3\text{-Br-}C_6H_4\text{-CH=CH-CH}_2\text{-}$ | CH | | |
| 188 | $3\text{-Br-}C_6H_4\text{-CH=CH-CH}_2\text{-}$ | N | | |
| 189 | $4\text{-Br-}C_6H_4\text{-CH=CH-CH}_2\text{-}$ | CH | E | Öl |
| 190 | $4\text{-Br-}C_6H_4\text{-CH=CH-CH}_2\text{-}$ | N | E | Öl |
| 191 | $2\text{-CH}_3\text{-}C_6H_4\text{-CH=CH-CH}_2\text{-}$ | CH | | |
| 192 | $2\text{-CH}_3\text{-}C_6H_4\text{-CH=CH-CH}_2\text{-}$ | N | | |
| 193 | $3\text{-CH}_3\text{-}C_6H_4\text{-CH=CH-CH}_2\text{-}$ | CH | | |

*) Stellung der Substituenten an der Doppelbindung $-C(COOCH_3)=X-OCH_3$

| Nr. | $R^1$ | X | Konfig.*) | physik. Daten (NMR in $CDCl_3$ [ppm]; TMS als Standard) |
|---|---|---|---|---|
| 194 | $3-CH_3-C_6H_4-CH=CH-CH_2-$ | N | | |
| 195 | $4-CH_3-C_6H_4-CH=CH-CH_2-$ | CH | | |
| 196 | $4-CH_3-C_6H_4-CH=CH-CH_2-$ | N | | |
| 197 | $2-CH_3O-C_6H_4-CH=CH-CH_2-$ | CH | | |
| 198 | $2-CH_3O-C_6H_4-CH=CH-CH_2-$ | N | | |
| 199 | $3-CH_3O-C_6H_4-CH=CH-CH_2-$ | CH | | |
| 200 | $3-CH_3O-C_6H_4-CH=CH-CH_2-$ | N | | |
| 201 | $4-CH_3O-C_6H_4-CH=CH-CH_2-$ | CH | | |
| 202 | $4-CH_3O-C_6H_4-CH=CH-CH_2-$ | N | | |
| 203 | $2-C_2H_5O-C_6H_4-CH=CH-CH_2-$ | CH | | |
| 204 | $2-C_2H_5O-C_6H_4-CH=CH-CH_2-$ | N | | |
| 205 | $3-C_2H_5O-C_6H_4-CH=CH-CH_2-$ | CH | | |
| 206 | $3-C_2H_5O-C_6H_4-CH=CH-CH_2-$ | N | | |
| 207 | $4-C_2H_5O-C_6H_4-CH=CH-CH_2-$ | CH | | |
| 208 | $4-C_2H_5O-C_6H_4-CH=CH-CH_2-$ | N | | |
| 209 | $4-(CH_3)_3C-C_6H_4-CH-CH=CH_2$ | CH | | |
| 210 | $4-(CH_3)_3C-C_6H_4-CH=CH-CH_2$ | N | | |
| 211 | $2-CF_3-C_6H_4-CH=CH-CH_2-$ | CH | | |
| 212 | $2-CF_3-C_6H_4-CH=CH-CH_2-$ | N | | |
| 213 | $3-CF_3-C_6H_4-CH=CH-CH_2-$ | CH | | |
| 214 | $3-CF_3-C_6H_4-CH=CH-CH_2-$ | N | | |
| 215 | $4-CF_3-C_6H_4-CH=CH-CH_2-$ | CH | | |
| 216 | $4-CF_3-C_6H_4-CH=CH-CH_2-$ | N | | |

*) Stellung der Substituenten an der Doppelbindung $-C(COOCH_3)=X-OCH_3$

| Nr. | $R^1$ | X | Konfig.*) | physik. Daten (NMR in $CDCl_3$ [ppm]; TMS als Standard) |
|---|---|---|---|---|
| 217 | $2,4-F_2-C_6H_3-CH=CH-CH_2-$ | CH | | |
| 218 | $2,4-F_2-C_6H_3-CH=CH-CH_2-$ | N | | |
| 219 | $2,6-F_2-C_6H_3-CH=CH-CH_2-$ | CH | | |
| 220 | $2,6-F_2-C_6H_3-CH=CH-CH_2-$ | N | | |
| 221 | $2,4-Cl_2-C_6H_3-CH=CH-CH_2-$ | CH | | |
| 222 | $2,4-Cl_2-C_6H_3-CH=CH-CH_2-$ | N | | |
| 223 | $2,6-Cl_2-C_6H_3-CH=CH-CH_2-$ | CH | | |
| 224 | $2,6-Cl_2-C_6H_3-CH=CH-CH_2-$ | N | | |
| 225 | $2-F-6-Cl-C_6H_3-CH=CH-CH_2-$ | CH | | |
| 226 | $2-F-6-Cl-C_6H_3-CH=CH-CH_2-$ | N | | |
| 227 | $3,4-Cl_2-C_6H_3-CH=CH-CH_2-$ | CH | | |
| 228 | $3,4-Cl_2-C_6H_3-CH=CH-CH_2-$ | N | | |
| 229 | $2,3,6-Cl_3-C_6H_2-CH=CH_2-CH_2-$ | CH | | |
| 230 | $2,3,6-Cl_3-C_6H_2-CH=CH_2-CH_2-$ | N | | |
| 231 | $2,4-(CH_3)_2-C_6H_3-CH=CH_2-CH_2-$ | CH | | |
| 232 | $2,4-(CH_3)_2-C_6H_3-CH=CH_2-CH_2-$ | N | | |
| 233 | $2,6-(CH_3)_2-C_6H_3-CH=CH_2-CH_2-$ | CH | | |
| 234 | $2,6-(CH_3)_2-C_6H_3-CH=CH_2-CH_2-$ | N | | |
| 235 | $3,4-(CH_3)_2-C_6H_3-CH=CH_2-CH_2-$ | CH | | |

*) Stellung der Substituenten an der Doppelbindung $-C(COOCH_3)=X-OCH_3$

| Nr. | $R^1$ | X | Konfig.*) | physik. Daten (NMR in $CDCl_3$ [ppm]; TMS als Standard) |
|---|---|---|---|---|
| 236 | $3,4-(CH_3)_2-C_6H_3-CH=CH_2-CH_2-$ | N | | |
| 237 | $2,4-(CH_3O)_2-C_6H_3-CH=CH_2-CH_2-$ | CH | | |
| 238 | $2,4-(CH_3O)_2-C_6H_3-CH=CH_2-CH_2-$ | N | | |
| 239 | $2,6-(CH_3O)_2-C_6H_3-CH=CH_2-CH_2-$ | CH | | |
| 240 | $2,6-(CH_3O)_2-C_6H_3-CH=CH_2-CH_2-$ | N | | |
| 241 | $3,4-(CH_3O)_2-C_6H_3-CH=CH_2-CH_2-$ | CH | | |
| 242 | $3,4-(CH_3O)_2-C_6H_3-CH=CH_2-CH_2-$ | N | | |
| 243 | $3,4-(C_2H_5O)_2-C_6H_3-CH=CH_2-CH_2-$ | CH | | |
| 244 | $3,4-(C_2H_5O)_2-C_6H_3-CH=CH_2-CH_2-$ | N | | |

*) Stellung der Substituenten an der Doppelbindung $-C(COOCH_3)=X-OCH_3$

| Nr. | $R^1$ | X | Konfig.*) | physik. Daten (NMR in $CDCl_3$ [ppm]; TMS als Standard) |
|---|---|---|---|---|
| 252 | $4-C_2H_5O-C_6H_4-$ | N | | |
| 253 | $2-CF_3-C_6H_4-$ | CH | | |
| 254 | $2-CF_3-C_6H_4-$ | N | | |
| 255 | $4-CF_3-C_6H_4-$ | CH | | |
| 256 | $4-CF_3-C_6H_4-$ | N | | |
| 257 | $2,4-(CH_3)_2-C_6H_3-$ | CH | | |
| 258 | $2,4-(CH_3)_2-C_6H_3-$ | N | | |
| 259 | $2,6-(CH_3)_2-C_6H_3-$ | CH | | |
| 260 | $2,6-(CH_3)_2-C_6H_3-$ | N | | |

*) Stellung der Substituenten an der Doppelbindung $-C(COOCH_3)=X-OCH_3$

Anwendungsbeispiele (fungizide Wirksamkeit)

Als Vergleichssubstanzen dienten

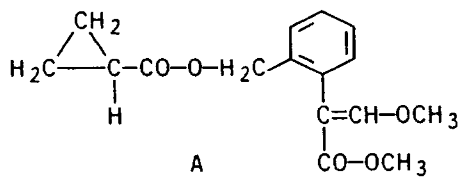
A

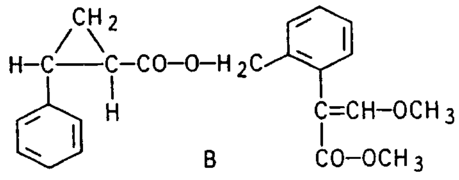
B

beide bekannt aus EP 310 954 (Verbindungen Nr. 71 und 364; beide E-Konfiguration an der Doppelbindung).

Beispiel 14

Wirksamkeit gegen Weizenmehltau

Blätter von in Töpfen gewachsenen Weizenkeimlingen der Sorte "Frühgold" wurden mit 0,006 und 0,0015 %igen wäßrigen Wirkstoffaufbereitungen, die 80 % Wirkstoff (der Wirkstoffe gemäß den Tabellenbeispielen 3, 4, 5, 6, 7, 15, und 18) und 20 % Emulgiermittel in der Trockenmasse enthielten, besprüht und 24 Stunden nach dem Antrocknen des Spritzbelages mit Sporen des Weizenmehltaus (Erysiphe graminis var. tritici) bestäubt. Die Versuchspflanzen wurden anschließend im Gewächshaus bei Temperaturen zwischen 20 und 22°C und 75 bis 80 % relativer Luftfeuchtigkeit aufgestellt. Nach 7 Tagen wurde das Ausmaß der Mehltauentwicklung beurteilt.

Gegenüber einem Kontrollversuch (keine Behandlung, 70 % Pilzbefall) und den bekannten Vergleichsverbindungen A (40-50 % Pilzbefall) und B (50 % Pilzbefall) zeigte sich, daß die behandelten Pflanzen nur einen Pilzbefall von 0-15 % hatten.

Beispiel 15

Wirksamkeit gegen Weizenbraunrost

Blätter von in Töpfen gewachsenen Weizensämlingen der Sorte "Frühgold" wurden mit Sporen des Braunrostes (Puccinia recondita) bestäubt. Danach wurden die Töpfe für 24 Stunden bei 20 bis 22°C in eine Kammer mit 90 bis 95 % relativer Luftfeuchtigkeit gestellt. Während dieser Zeit keimten die Sporen aus und die Keimschläuche drangen in das Blattgewebe ein. Die infizierten Pflanzen wurden mit 0,025 %iger wäßriger Spritzbrühe, die 80 % Wirkstoff und 20 % Emulgiermittel in der Trockensubstanz enthielten, tropfnaß gespritzt und nach dem Antrocknen des Spritzbelages im Gewächshaus bei einer Temperatur zwischen 20 und 22°C und 65 bis 70 % relativer Luftfeuchtigkeit aufgestellt. Nach 8 Tagen beurteilte man das Ausmaß der Rostpilzentwicklung auf den Blättern.

Das Ergebnis zeigt, daß die Wirkstoffe 3, 5, 7, 15, 24, 45, 103, 109, 110 und 177 bei der Anwendung als 0,025 %ige (Gew.-%) Spritzbrühe eine bessere fungizide Wirkung aufweisen (97 %) als die bekannten Vergleichssubstanzen A (33 %) und B (17 %).

Beispiel 16

Wirksamkeit gegen Pyricularia oryzae (vorbeugende Behandlung)

Blätter von in Töpfen gewachsenen Reiskeimlingen der Sorte "Bahia" wurden mit wäßrigen Emulsionen, die 80 % Wirkstoff und 20 % Emulgiermittel in der Trockensubstanz enthielten, tropfnaß besprüht und 24 Stunden später mit einer wäßrigen Sporensuspension von Pyricularia oryzae infiziert. Die Versuchspflanzen wurden anschließend in Klimakammern bei Temperaturen zwischen 20 und 24°C und 95-99 % relativer Luftfeuchtigkeit aufgestellt. Nach 6 Tagen wurde das Ausmaß des Pilzbefalls ermittelt.

Das Ergebnis zeigt, daß die Wirkstoffe 3, 4, 5, 6, 8, 46, 103, 104, 109, 177, und 183 bei der Anwendung als 0,05 %ige (Gew.-%) wäßrige Wirkstoffaufbereitung eine viel bessere fungizide Wirkung aufweisen (98

%) als die bekannten Vergleichssubstanzen A (0 %) und B (50 %).

Anwendungsbeispiele (insektizide Wirksamkeit)

Die insektizide Wirkung der ortho-substituierten Benzylester von Cyclopropancarbonsäuren I ließ sich durch folgende Versuche zeigen:

Die Wirkstoffe wurden als 10 %ige Emulsion in einem Gemisch aus 70 Gew.-% Cyclohexanol, 20 Gew.-% Netzmittel mit Emulgier- und Dispergierwirkung auf Basis ethoxylierter Alkylphenole und 10 Gew.-% Emulgator auf Basis ethoxylierter Fettalkohole aufbereitet und entsprechend der gewünschten Konzentration mit Wasser verdünnt.

Beispiel 17

Wirksamkeit gegen Tetranychus telarius (Rote Spinne); Kontaktwirkung

Versuch 17 a

In Töpfen befindliche Buschbohnen, die das zweite Folgeblattpaar gebildet hatten und die stark mit den Spinnmilben befallen waren, wurden mit verschieden konzentrierten wäßrigen Wirkstoffaufbereitungen der Verbindung Nr. 29 tropfnaß gespritzt. Dazu besprühte man die Pflanzen auf einem Drehteller von allen Seiten mit ca. 50 ml der Spritzbrühe. Nach 5 Tagen im Gewächshaus wurde mittels Mikroskop (Binokular) der Bekämpfungserfolg in % festgestellt.

Bei einer Wirkstoffkonzentration von 200 ppm bewirkte die Verbindung Nr. 29 eine Mortilität von 80 bis 90 %.

Versuch 17 b (vorbeugende Behandlung)

In Töpfen befindliche Buschbohnen, die das zweite Folgeblattpaar gebildet hatten, wurden mit verschieden konzentrierten Wirkstoffaufbereitungen der Verbindung Nr. 29 analog Versuch 17a tropfnaß gespritzt. Nach 24 Stunden wurden die Pflanzen mit Blattstücken, die stark mit Spinnmilben besetzt waren, infiziert.

Nach 12 Tagen im Gewächshaus wurde mittels Mikroskop (Binokular) der Bekämpfungserfolg in % festgestellt.

Bei einer Wirkstoffkonzentration von 40 ppm bewirkte die Verbindung Nr. 29 eine Mortalität von 80 bis 90 %.

## Patentansprüche

**1.** Ortho-substituierte Benzylester von Cyclopropancarbonsäuren der allgemeinen Formel I

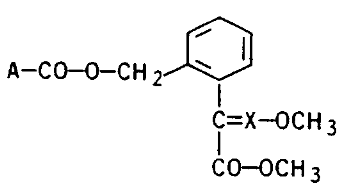

I

in der X für Stickstoff oder =CH- und A für den folgenden Cyclopropanrest steht:

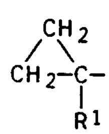

,

wobei der Substituent $R^1$ die folgende Bedeutung hat:

Cyano, $C_2$-$C_8$-Alkyl, Trifluormethyl, $C_3$-$C_8$-Alkenyl, Trimethylsilyl, Phenyl-$C_1$-$C_6$-alkyl oder Phenyl$C_3$-$C_6$-alkenyl, wobei der Aromat jeweils noch 1-5 Halogenatome oder bis zu 3 der folgenden

Substituenten tragen kann: $C_1$-$C_6$-Alkyl, partiell oder vollständig halogeniertes $C_1$-$C_6$-Alkyl oder $C_1$-$C_6$-Alkoxy, $R^1$ bedeutet außerdem noch 2-Bromphenyl, 4-Trifluormethylphenyl, 2,4-Difluorphenyl, 2,6-Difluorphenyl oder 2-Fluor-6-chlorphenyl; mit der Maßgabe, daß X für =CH- steht, wenn $R^1$ Trifluormethyl oder Trimethylsilyl bedeutet.

**2.** Ortho-substituierte Benzylester von Cyclopropancarbonsäuren der allgemeinen Formel Ia

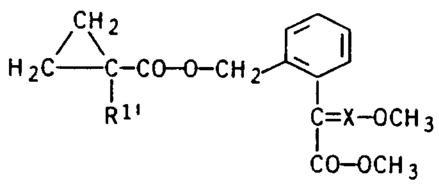

Ia

in der X für Stickstoff oder =CH- steht und $R^{1''}$ folgende Bedeutung hat:

Cyano, $C_2$-$C_4$-Alkyl, Trifluormethyl, $C_3$-$C_4$-Alkenyl, Trimethylsilyl, Phenyl-$C_1$-$C_4$-alkyl oder Phenyl-$C_3$-$C_4$-alkenyl, wobei der Aromat jeweils noch durch Halogen oder Methyl einfach substituiert sein kann, 2-Bromphenyl, 2,4-Difluorphenyl, 2,6-Difluorphenyl und 2-Fluor-6-chlorphenyl, mit der Maßgabe, daß X für =CH- steht, wenn $R^{1''}$ Trimethylsilyl oder Trifluormethyl bedeutet.

**3.** Verfahren zur Herstellung der ortho-substituierten Benzylester von Cyclopropancarbonsäuren I gemäß Anspruch 1, dadurch gekennzeichnet, dap man eine Cyclopropancarbonsäure der Formel II

A-CO-OH II

in ihr Alkalimetallsalz, Erdalkalimetallsalz, Ammoniumsalz, $C_1$-$C_4$-Alkylammonium-, Di-($C_1$-$C_4$)-alkylammonium- oder Tri-($C_1$-$C_4$)-alkylammoniumsalz überführt und dieses Salz, gewünschtenfalls in Gegenwart eines Katalysators, mit einer ortho-substituierten Benzylverbindung der Formel III

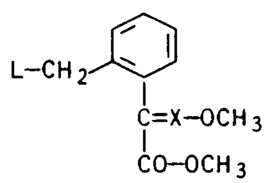

III

in der L für eine nukleophil substituierbare Abgangsgruppe steht, umsetzt.

**4.** Fungizides Mittel, enthaltend einen flüssigen oder festen Trägerstoff und einen ortho-substituierten Benzylester von Cyclopropancarbonsäuren der Formel I gemäß Anspruch 1.

**5.** Schädlingsbekämpfungsmittel, enthaltend einen inerten Trägerstoff und einen ortho-substituierten Benzylester von Cyclopropancarbonsäuren der Formel I gemäß Anspruch 1.

**6.** Verfahren zur Bekämpfung von Pilzen, dadurch gekennzeichnet, daß man eine fungizid wirksame Menge eines ortho-substituierten Benzylesters von Cyclopropancarbonsäuren der Formel I gemäß Anspruch 1. auf Pilze, vom Pilzbefall bedrohte Pflanzen, deren Lebensraum oder auf das Saatgut der bedrohten Pflanzen einwirken läßt.

**7.** Verfahren zur Bekämpfung von Schädlingen, dadurch gekennzeichnet, daß man eine pestizid wirksame Menge eines ortho-substituierten Benzylesters von Cyclopropancarbonsäuren der Formel I gemäß Anspruch 1 auf Schädlinge bzw. ihren Lebensraum einwirken läßt.

**8.** Verbindung der Formel I gemäß Anspruch 1, in der $R^1$ n-Propyl und X CH bedeutet.

**9.** Verbindung der Formel I gemäß Anspruch 1, in der $R^1$ n-Propyl und X N bedeutet.

**10.** Verbindung der Formel I gemäß Anspruch 1, in der $R^1$ Allyl und X CH bedeutet.

**Claims**

**1.** An ortho-substituted benzyl ester of a cyclopropanecarboxylic acid of the formula I

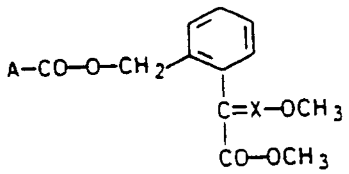

I

where X is nitrogen or =CH- and A is the following cyclopropane radical:

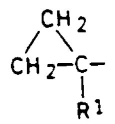

,

where $R^1$ is cyano, $C_2$-$C_8$-alkyl, trifluoromethyl, $C_3$-$C_8$-alkenyl, trimethylsilyl, phenyl-$C_1$-$C_6$-alkyl or phenyl-$C_3$-$C_6$-alkenyl, where each aromatic moiety may furthermore carry 1-5 halogen atoms or up to 3 of the following substituents: $C_1$-$C_6$-alkyl, partially or completely halogenated $C_1$-$C_6$-alkyl or $C_1$-$C_6$-alkoxy, or is 2-bromophenyl, 4-trifluoromethylphenyl, 2,4-difluorophenyl, 2,6-difluorophenyl or 2-fluoro-6-chlorophenyl, with the proviso that X is =CH- when $R^1$ is trifluoromethyl or trimethylsilyl.

**2.** An ortho-substituted benzyl ester of a cyclopropanecarboxylic acid of the formula Ia

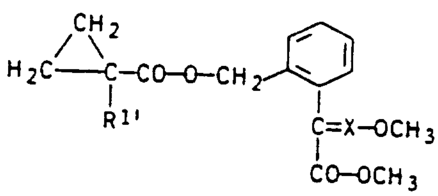

Ia

where X is nitrogen or =CH- and $R^{1'}$ is cyano, $C_2$-$C_4$-alkyl, trifluoromethyl, $C_3$- or $C_4$-alkenyl, trimethylsilyl, phenyl-$C_1$-$C_4$-alkyl or phenyl-$C_3$- or -$C_4$-alkenyl, where each aromatic moiety may furthermore be monosubstituted by halogen or methyl, 2-bromophenyl, 2,4-difluorophenyl, 2,6-difluorophenyl or 2-fluoro-6-chlorophenyl,
with the proviso that X is =CH- when $R^{1'}$ is trimethylsilyl or trifluoromethyl.

**3.** A process for the preparation of an ortho-substituted benzyl ester of a cyclopropanecarboxylic acid I as claimed in claim 1, wherein a cyclopropanecarboxylic acid of the formula II

A-CO-OH II

is converted into its alkali metal salt, alkaline earth metal salt, ammonium salt or $C_1$-$C_4$-alkylammonium, di-$C_1$-$C_4$-alkylammonium or tri-$C_1$-$C_4$-alkylammonium salt, and this salt is reacted, if desired in the presence of a catalyst, with an ortho-substituted benzyl compound of the formula III

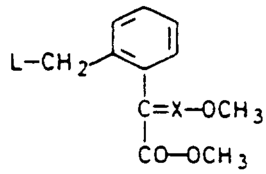

III

where L is a nucleophilically substitutable leaving group.

**4.** A fungicide containing a liquid or solid carrier and an ortho-substituted benzyl ester of a cyclopropanecarboxylic acid of the formula I as claimed in claim 1.

**5.** A pesticide containing an inert carrier and an ortho-substituted benzyl ester of a cyclopropanecarboxylic acid of the formula I as claimed in claim 1.

**6.** A method for controlling fungi, wherein a fungicidal amount of an ortho-substituted benzyl ester of a cyclopropanecarboxylic acid of the formula I as claimed in claim 1 is allowed to act on fungi, on plants threatened by fungal attack, on their habitat or on the seed of the threatened plants.

**7.** A method for controlling pests, wherein a pesticidal amount of an ortho-substituted benzyl ester of a cyclopropanecarboxylic acid of the formula I as claimed in claim 1 is allowed to act on pests or their habitat.

**8.** A compound of the formula I as claimed in claim 1, where $R^1$ is n-propyl and X is CH.

**9.** A compound of the formula I as claimed in claim 1, where $R^1$ is n-propyl and X is N.

**10.** A compound of the formula I as claimed in claim 1, where $R^1$ is allyl and X is CH.

## Revendications

**1.** Ester benzylique, substitué en ortho, d'acide cyclopropane-carboxylique de la formule générale I

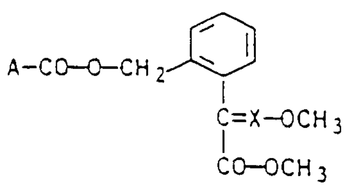

I

dans laquelle X est mis pour azote ou =CH- et A pour le reste cyclopropane suivant :

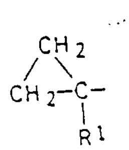

le substituant $R^1$ ayant la signification suivante :
cyano, alkyle en C2-C8, trifluorométhyle, alcényle en C3-C8, triméthylsilyle, phényle-alkyle en C1-C8 ou phényl-alcényle en C3-C6, l'aromate pouvant porter chaque fois encore 1-5 atomes d'halogène ou jusqu'à 3 des substituants suivants :
alkyle en C1-C6, alkyle en C1-C4 ou alcoxy en C1-C6 partiellement ou totalement halogéné, R1 signifie en outre encore, 2-bromophényle, 4-trifluorométhylphényle, 2,4-difluorophényle, 2,6-difluorophényle ou 2-fluoro-6-chlorophényle
sous réserve que X soit mis pour =CH- quand $R^1$ représente trifluorométhyle ou triméthylsilyle.

2. Ester benzylique, substitué en ortho, d'acide cyclopropanecarboxylique de la formule générale Ia

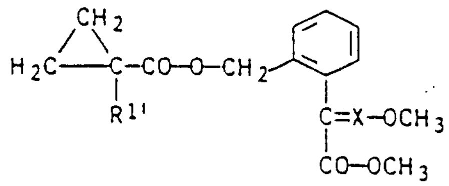

Ia

dans laquelle X est mis pour azote ou =CH- et $R^{1'}$ a la signification suivante :
cyano, alkyle en C2-C4, trifluorométhyle, alcényle en C3-C4, triméthyl-silyle, phényle-alkyle en C1-C4 ou phényl-alcényle en C3-C4, l'aromate pouvant être chaque fois, encore substitué une fois par halogène ou méthyle, 2-bromophényle, 2,4-difluorophényle, 2,6-difluorophényle et 2-fluoro-6-chlorophényle
sous réserve que X soit mis pour =CH- quand $R^1$ représente trifluorométhyle ou triméthylsilyle.

3. Procédé de préparation d'ester benzylique, substitué en ortho, d'acide cyclopropane-carboxylique de la formule générale I selon la revendication I, caractérisé par le fait que l'on transforme un acide cyclopropanecarboxylique de la formule II

A-CO-OH    II

en son sel de métal alcalin, sel de métal alcalino-terreux, sel d'ammonium, sel d'alkyle en C1-C4-ammonium, sel de di-(alkyle en C1-C4)-ammonium ou sel de tri-(alkyle en C1-C4)-ammonium et on fait réagir ce sel, éventuellement en présence d'un catalyseur, avec un composé benzylique substituée en ortho, de la formule III

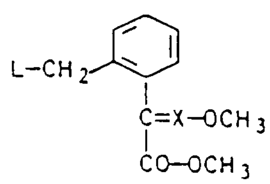

III

dans laquelle L est mis pour un groupe éliminable substituable par nucléophile.

4. Fongicide contenant un support liquide ou solide et un ester benzylique, substitué en ortho, d'acide cyclopropane carboxylique de la formule générale I selon la revendication 1.

5. Agent de lutte contre les parasites contenant un support liquide ou solide et un ester benzylique, substitué en ortho, d'acide cyclopropane carboxylique de la formule générale I selon la revendication 1.

6. Procédé de lutte contre les champignons, caractérisé par le fait que l'on fait agir sur les champignons, sur les plantes menacés par les champignons, sur leur biotope ou sur les semences des plantes menacés d'une attaque par les champignons, une quantité efficace au point de vue fongicide d'un ester benzylique, substitué en ortho, d'acide cyclopropane carboxylique de la formule générale I selon la revendication 1.

7. Procédé de lutte contre les parasites, caractérisé par le fait que l'on fait agir sur les parasites ou leur biotope une quantité efficace au point de vue pesticide d'un ester benzylique, substitué en ortho, d'acide cyclopropane carboxylique de la formule générale I selon la revendication 1.

8. Composé de formule I selon la revendication 1 dans laquelle $R^1$ représente n-propyle et X, CH.

9. Composé de formule I selon la revendication 1 dans laquelle $R^1$ représente n-propyle et X, N.

**10.** Composé de formule I selon la revendication 1 dans laquelle $R^1$ représente alkyle et X, CH.